# EUROPEAN PATENT APPLICATION

(11) **EP 3 231 875 A1**
(43) Date of publication of application: **18.10.2017**
(21) Application number: 16382173.9
(22) Date of filing: 15.04.2016
(51) Int. Cl.: C12Q 1/68

(54) **PROGNOSTIC BIOMARKER FOR CANCER**

(71) Applicant: Universitat Autònoma de Barcelona, 08193 Barcelona (ES); Fundació Institut de Recerca Biomèdica de Bellvitge, 08907 Hospitalet del Llobregat-Barcelona (ES); Institut Català D'Oncologia, 08907 L'Hospitalet de Llobregat (ES)
(72) Inventor: SURRALLÉS CALONGE, Jordi, E-08193 Bellaterra - Barcelona (ES); MINGUILLON PEDREÑO, Jordi, E-08193 Bellaterra - Barcelona (ES); GENESTAR PUJANA, Miguel Ángel, E-08908 Hospitalet de Llobregat - Barcelona (ES); LAZARO GARCÍA, Concepción, E-08908 Hospitalet de Llobregat - Barcelona (ES)
(74) Representative: ABG Patentes, S.L.

(57) **Abstract**

The invention relates to an *in vitro* method for determining the prognosis of a patient suffering from cancer, wherein the cancer is not hepatocarcinoma, based on CDK5RAP3 gene expression levels. The invention also relates *to in vitro* methods for predicting the clinical response of a patient suffering from cancer to a treatment with a DNA damaging agent, or for selecting a patient suffering from cancer for a therapy with a DNA damaging agent or for selecting a therapy for a patient suffering from cancer.

## Description

### FIELD OF THE INVENTION

The invention relates to the field of biomarkers for the prognostic of cancer. In particular, the invention relates to a method for determining the prognosis of a patient suffering from cancer, or for predicting the clinical response of said patient to a treatment with a DNA damaging agent, or for selecting a cancer patient for a therapy with a DNA damaging agent.

### BACKGROUND OF THE INVENTION

Breast cancer is the second most common type of cancer worldwide and the fifth most common cause of death by cancer (after lung cancer, stomach cancer, liver cancer, and colon cancer). Among women, breast cancer is the most common cause of death by cancer.

Breast cancer is classified into stages according to the TNM system. The prognosis is closely related to the results of the stage classification, and the stage classification is also used to assign patients to treatments both in clinical trials and in the medical practice. The information for classifying into stages is as follow:
There are three significant receptors which may affect the breast cancer cells: estrogen receptor (ER), progesterone receptor (PR) and HER2/neu. The receptor state has become a critical assessment for all breast cancers since it determines the suitability of using specific treatments, for example, tamoxifen or trastuzumab. The alpha isoform of the estrogen receptor (ER) is over-expressed in about 65% of the diagnosed cases of breast cancer. This type of breast cancer is referred to as "ER-positive" (ER+). In this case the binding of the estrogen to the ER stimulates the tumor mammary cell proliferation. The ER+ tumor cells are highly dependent on this stimulus to proliferate, therefore ER is currently used as a therapeutic target.

Today no set of predictors of a satisfactory prognosis based solely on clinical information has been identified. Over the last 30 years oncologists have focused on optimizing the outcome of the cancer patients and it is only now that the new available technologies allow investigating polymorphisms, expression levels of genes and gene mutations for the purpose of predicting the impact of a determined therapy on different groups of cancer patients in order to design customized chemotherapies. PCR assays such as Oncotype DX or microarray assays such as MammaPrint can predict the risk of breast cancer relapse based on gene expression. In February 2007, the MammaPrint assay became the first breast cancer indicator to receive official authorization from the Food and Drug Administration.

Ovarian cancer is the gynecological disease causing the highest number of deaths in developed countries. With an incidence of 204,000 cases a year, it causes 125,000 deaths worldwide. The 5-year mortality is about 70-80%, in most cases being due to tumor progression and metastasis.

The most common type of ovarian cancer is ovarian epithelial cancer (90%). Epithelial tumors include benign tumors, low malignant potential tumors or borderline tumors and malignant tumors, the prognosis of which depends fundamentally on the stage (I-III) (Ovarian Cancer Detailed Guideline, ACS 2013). Like in other tumors, surgical and pharmacological treatment suitable for each type of tumor depends on the spread of the tumor and on its risk of progression; but in ovarian cancer surgery decision must be the most conservative possible in order to prevent loss of fertility in patients. For this reason, it is of vital importance to correctly classify the tumors and evaluate the tumor progression and metastasis capacity at the time of diagnosis, which would allow selecting the correct surgical procedure for each patient.

Therefore, there is a need to develop new methods which allow predicting the prognosis of a patient suffering from cancer in general, and breast and ovarian cancer in particular, more efficiently than the methods described in the state of the art. The identification of new prognosis factors will serve as a guideline in selecting the most suitable treatments.

### BRIEF DESCRIPTION OF THE INVENTION

The inventors of the present invention have discovered that, surprisingly, CDK5RAP3 depletion results in resistance to DNA damaging drugs and ionizing radiation (Fig. 2) and that low levels of CDK5RAP3 in breast and ovarian tumors strongly associate with lower survival (Fig. 8 and 9). Therefore, CDK5RAP3 is a novel biomarker of tumor aggressiveness with prognostic value.

Therefore, in one aspect the invention relates to an *in vitro* method for determining the prognosis of a patient suffering from cancer, wherein the cancer is not hepatocarcinoma, comprising the steps of:
(a) determining the expression level of Cyclin-Dependent Kinase 5 Regulatory Subunit Associated Protein 3 (CDK5RAP3) gene in a sample from said patient, and
(b) comparing said expression level with a reference value,
wherein a decreased expression level of CDK5RAP3 gene compared to the reference value is indicative of a poor prognosis or
wherein an increased expression level of CDK5RAP3 gene or a lack of change compared to the reference value is indicative of a good prognosis.

In another aspect, the invention relates to an *in vitro* method for predicting the clinical response of a patient suffering from cancer to a treatment with a DNA damaging agent comprising the steps of:
(a) determining the expression level of CDK5RAP3 gene in a sample from said patient and
(b) comparing said expression level with a reference value,
wherein a decreased expression level of CDK5RAP3 gene compared to the reference value is indicative of a poor clinical response of the patient to said treatment.

In another aspect, the invention relates to an *in vitro* method for selecting a patient suffering from cancer for a therapy with a DNA damaging agent, comprising the steps of:
(a) determining the expression level of CDK5RAP3 gene in a sample from said patient and
(b) comparing said expression level with a reference value,
wherein the patient is selected for said therapy if the expression level of CDK5RAP3 gene is not decreased compared to the reference value.

In another aspect, the invention relates to an *in vitro* method for selecting a therapy for a patient suffering from cancer, comprising the steps of
(a) determining the expression level of CDK5RAP3 gene in a sample from said patient and
(b) comparing said expression level with a reference value,
wherein if the expression level of CDK5RAP3 gene is not decreased compared to the reference value, a therapy with a DNA damaging agent is selected and
wherein if the expression level of CDK5RAP3 gene is decreased compared to the reference value, an alternative therapy is selected.

In another aspect, the invention relates to the use of a reagent capable of determining the expression level of CDK5RAP3 gene in a sample from a subject suffering from cancer for determining the prognosis of said patient, wherein the cancer is not hepatocarcinoma, or for predicting the clinical response of a patient suffering from cancer to a treatment with a DNA damaging agent, or for selecting a patient suffering from cancer for a therapy with a DNA damaging agent, or for selecting a therapy for a patient suffering from cancer.

In another aspect, the invention relates to a DNA damaging agent-based treatment for use in the treatment of cancer in a patient, wherein a sample of said patient shows increased or equal expression levels of CDK5RAP3 gene compared to a reference value.

### BRIEF DESCRIPTION OF THE FIGURES

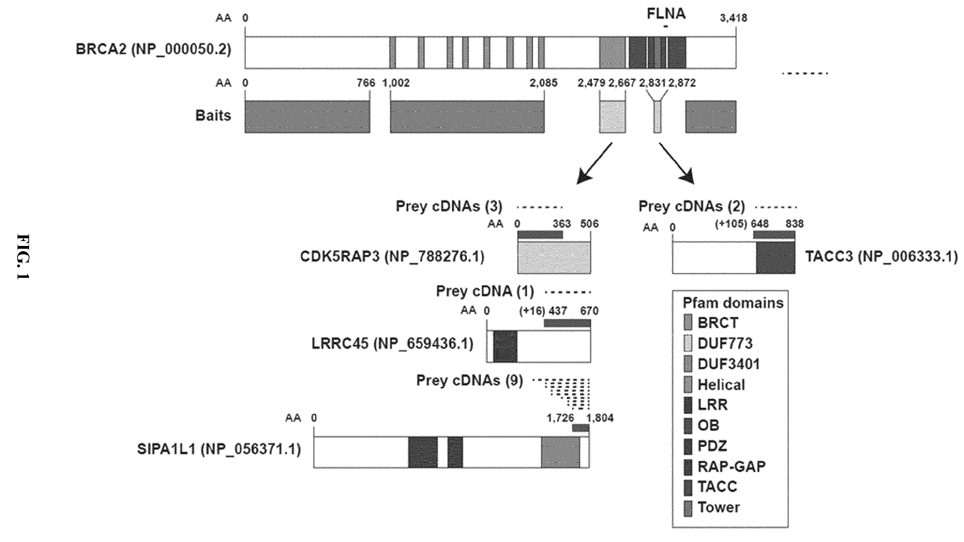

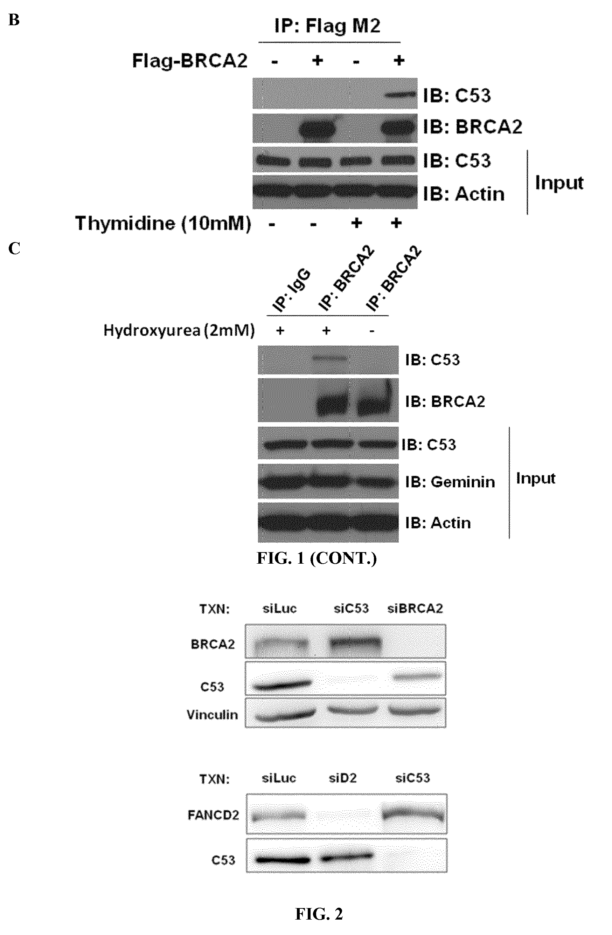

**Figure 1****. C53 interacts with BRCA2.** A) BRCA2 fragments used as baits for yeast-two-hybrid assay and potential interactors found (see materials and methods). B) HEK 293T cells expressing empty vector or Flag-BRCA2 were treated with double thymidine block (10 mM) followed by a 9 hours release to enrich S-phase cells, or left untreated. Cells were lysed and subjected to immunoprecipitation (IP) with anti-Flag M2 antibody. Immunoprecipitates and whole cell lysates were immunoblotted (IB) with antibodies shown. C) HEK 293T cells were left untreated or synchronised to S-phase by treatment with 2 mM hydroxyurea for 18 hours. Cells were lysed and subjected to IP with anti-IgG or anti-BRCA2 antibody, followed by immunoblotting with antibodies shown. AA, aminoacid; FLNA, filamin A, alpha.

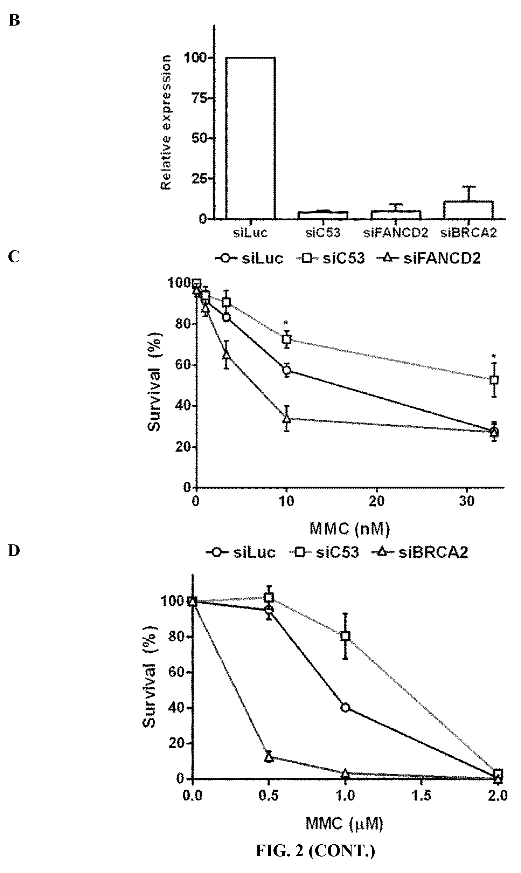

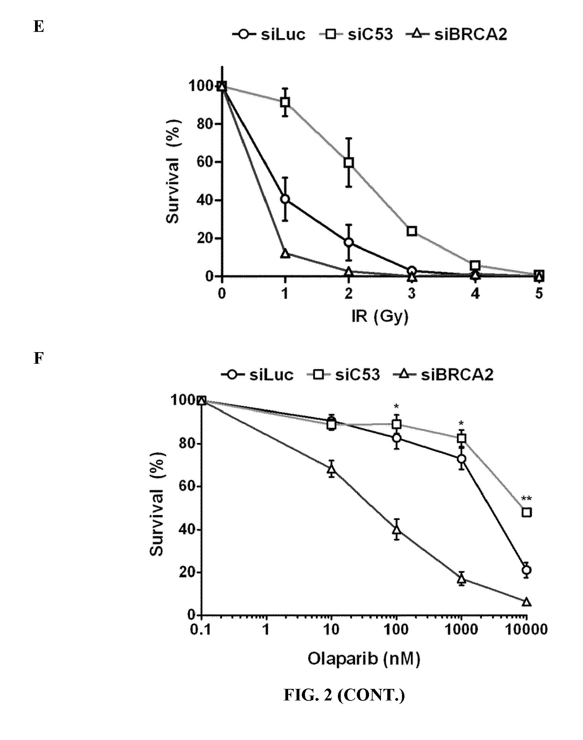

**Figure 2****. Lack of C53 promotes resistance to DNA damaging agents.** A) For survival assays, U2OS cells were transfected with siRNA targeting C53, BRCA2, FANCD2 or luciferase as a control (see materials and methods). Once counted, non-stimulated cells were lysed and western blot was performed with the indicated antibodies. B) Graph quantification of western blots performed as in A, showing mean ± SD of at least three independent survival experiments. A reduction of more than 90% expression in all 3 proteins was obtained. C, F) U2OS cells were transfected with siLuc, siC53, siFANCD2 or siBRCA2. 24 hours post-transfection cells were plated and treated with different MMC (C) or Olaparib (F) doses for 3-5 days, then collected and counted (survival assays). For clonogenic assays, cells were plated and stimulated with different MMC (D) or IR (E) doses for 10-14 days, Giemsa stained and colonies counted. Survival assays were done at least three times with duplicates or triplicates, and clonogenic assays at least two times with duplicates. TXN, transfection; MMC, mitomycin C; IR, γ-radiation. *p<0.05, **p<0.01.

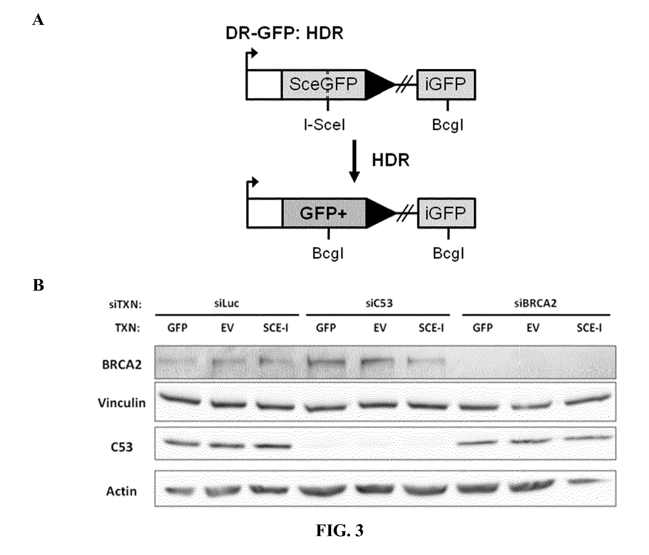

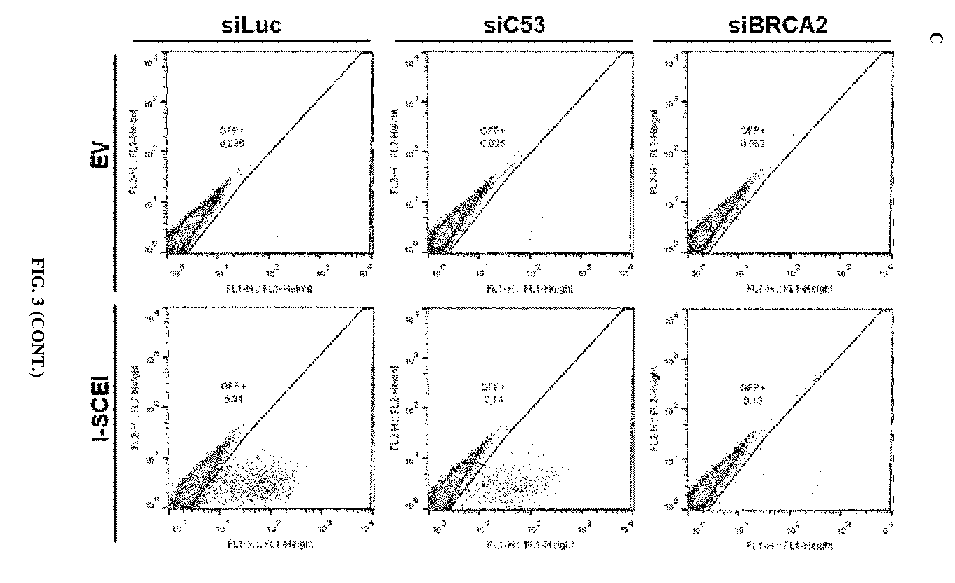

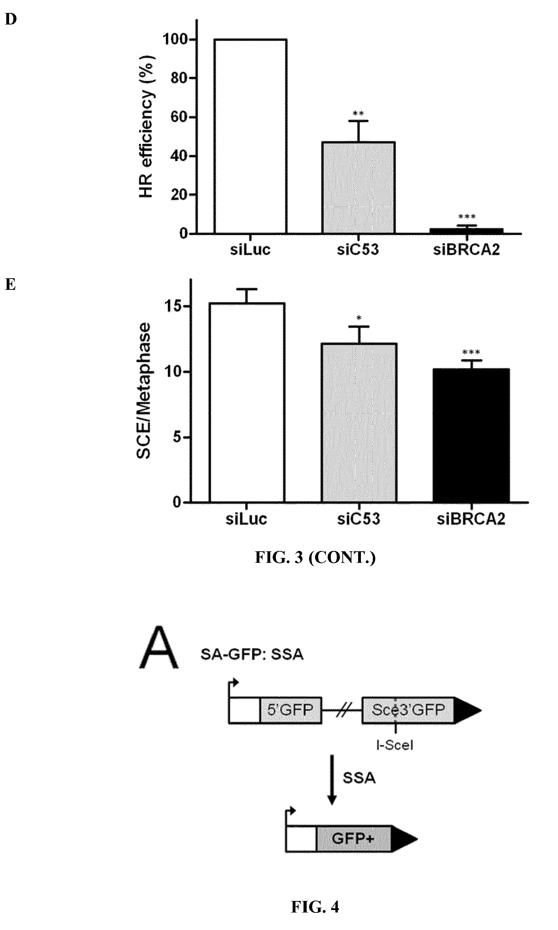

**Figure 3****. C53 is required for DNA repair by homologous recombination.** A) Diagram showing the *in vivo* homologous directed repair (HDR) assay. U2OS cells carry a plasmid inserted in the genome with two adjacent non-functional GFP coding fragments, one of them with an I-SceI restriction site. When a plasmid expressing I-SceI endonuclease is transfected, a specific DSB is generated in one GFP coding fragment, promoting recombination with the adjacent fragment and the formation of a full, functional GFP coding sequence resulting in fluorescent cells. B) DR-GFP stable U2OS cells were transfected with siLuc, siC53 or siBRCA2. 24h later, cells were transfected with GFP, I-SceI expressing plasmids or empty vector and 24-48h later collected and lysed for western blot analysis with the indicated antibodies. C) Flow cytometry plots from Figure 3B Cells. D) Averaged graph from at least three independent experiments as in C. GFP transfected cells were used to normalize transfection efficiency. E) Sister chromatid exchange (SCE) analysis in primary fibroblasts (see materials and methods). Graph shows analysis of 15 metaphases on average from siRNA transfections. Bars show mean ± SEM. *p<0.05 **p<0.01 ***p<0.001.

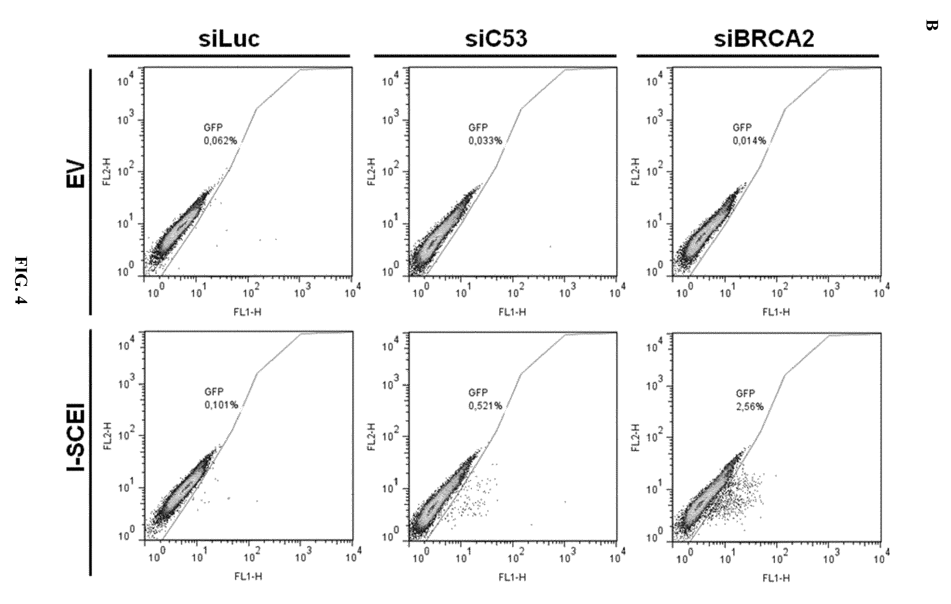

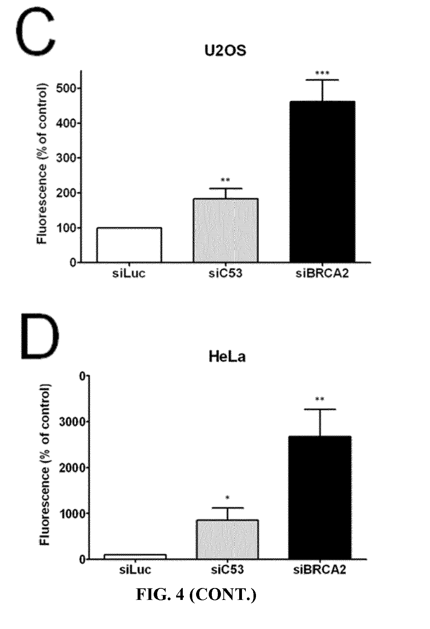

**Figure 4****. C53 regulates single strand annealing repair in a BRCA2 similar manner.** A) Diagram showing the *in vivo* single strand annealing (SSA) repair assay. U2OS or HeLa cells carry inserted in the genome a plasmid with two adjacent non-functional GFP coding fragments, one short 5' fragment and a 3' fragment one with an I-SceI restriction site. When a plasmid expressing I-SceI endonuclease is transfected, a specific DSB is generated in the 3' GFP coding fragment, and a full, functional GFP coding sequence is formed after strand annealing homolog searching and fusion with the 5' GFP fragment. B) SA-GFP stable HeLa cells were transfected with siLuc, siC53 or siBRCA2. After 24h cells were transfected with GFP, I-SceI expressing plasmids or empty vector and 24-48h later fluorescence was analysed by flow cytometry. C-D) Averaged graph from at least three independent experiments as in B were performed in U2OS (C) or Hela (D) cells. GFP transfected cells were used to normalize transfection efficiency. Bars show mean ± SEM. **p<0.01 ***p<0.001.

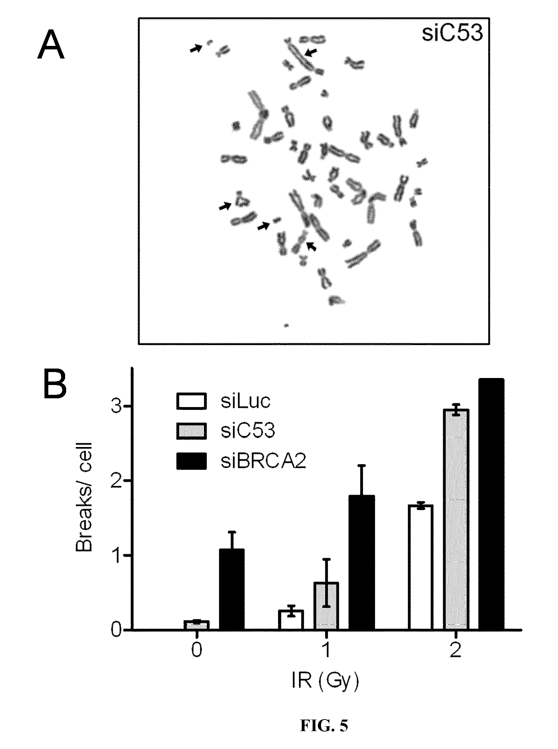

**Figure 5****. C53 inhibition induces spontaneous and IR-induced genomic instability.** Primary fibroblasts were transfected with siRNA targeting C53, BRCA2 or luciferase as a control. 24 hours post-transfection cells were plated and irradiated at different doses, and 72 hours later metaphases were analysed for chromosome fragility (see materials and methods). A) Representative metaphase of siC53-transfected and 1 Gy-irradiated primary fibroblasts, showing trirradial and dicentric chromosomes, as well as chromosome breaks. B) Cell metaphases were counted for chromosome breaks. Graph shows breaks/cell mean ± SD of two independent experiments.

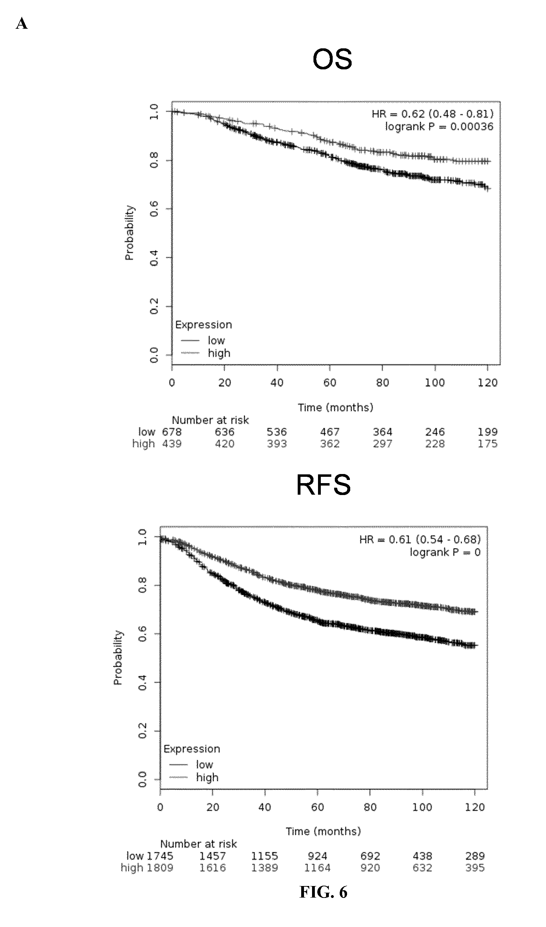

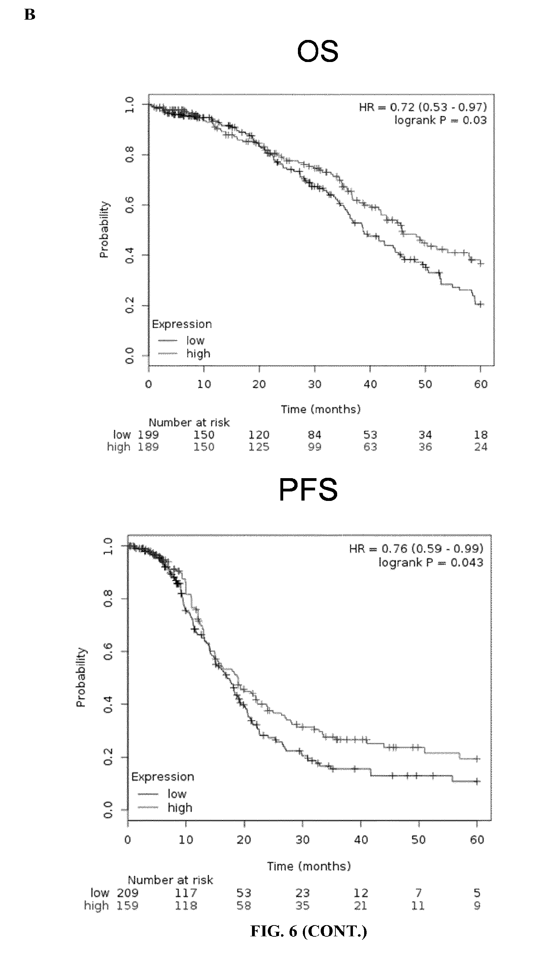

**Figure 6****. Low C53 expression correlates with poor patient survival in breast and ovarian cancer datasets.** A) Kaplan-Meier survival curves for 3554 (RFS, Relapse Free Survival) or 1117 (OS, Overall Survival) patients with breast cancer. Data were divided at the best cutoff value into high and low expressing groups (for RFS, *n* = 1745 for C53 low, *n* = 1809 for C53 high; *P* = 0 with log-rank analysis; for OS, *n* = 678 for C53 low, *n* = 439 for C53 high; *P* = 0.00036 with log-rank analysis). B) Kaplan-Meier survival curves for 368 (PFS, Progression Free Survival) or 388 (OS, Overall Survival) patients with high grade serous ovarian cancer (HGSOC). Data were divided at the best cutoff value into high and low expressing groups (for PFS, *n* = 209 for C53 low, *n* = 159 for C53 high; *P* = 0.043 with log-rank analysis; for OS, *n* = 199 for C53 low, *n* = 189 for C53 high; *P* = 0.03 with log-rank analysis). Data obtained from the Kaplan Meier plotter (http://www.kmplot.com).

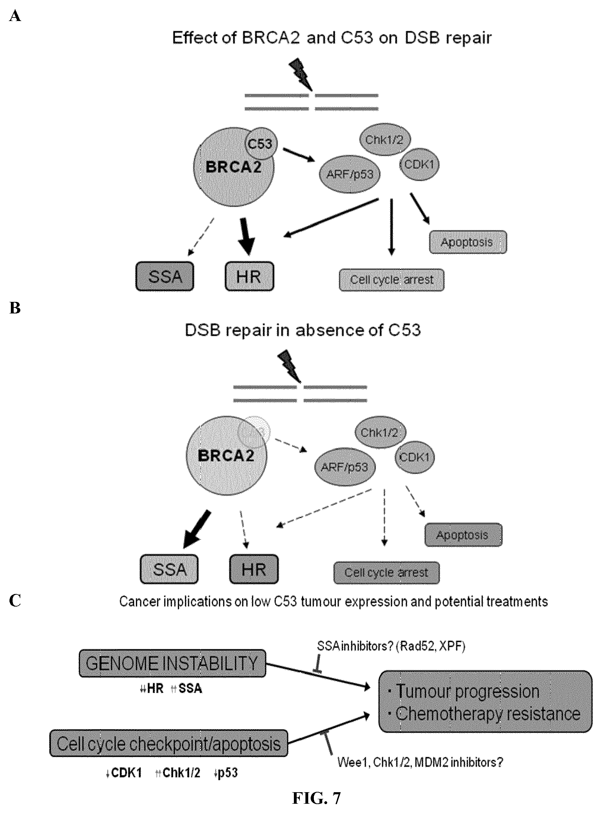

**Figure 7****. Model of C53 role in DNA repair and cancer.** A) Effect of BRCA2 and C53 on DSB repair. In the presence of normal C53 and BRCA2 levels, DSB induced damage is repaired in S-phase by HR, with balanced control of cell cycle arrest and apoptosis by p53, Chk 1/2 proteins and cyclin dependent kinases. B) DSB repair in absence of C53. C53 low expression reduces HR repair counteracted by an increase in SSA. At the same time, apoptosis control is imbalanced, leading to DNA damage resistant cells. C) Cancer implications on low C53 tumor expression and potential treatments. C53 absence increases genome instability and imbalances apoptosis, promoting tumor progression and chemotherapy resistance. Blocking SSA repair with Rad52/XPF inhibitors may counteract the DNA damage resistance, and restoring DNA damage sensitivity by Weel, Chkl/2 or MDM2 inhibitors could rescue apoptosis induction.

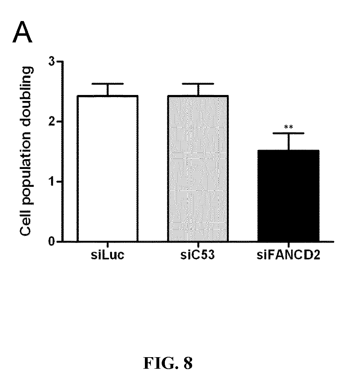

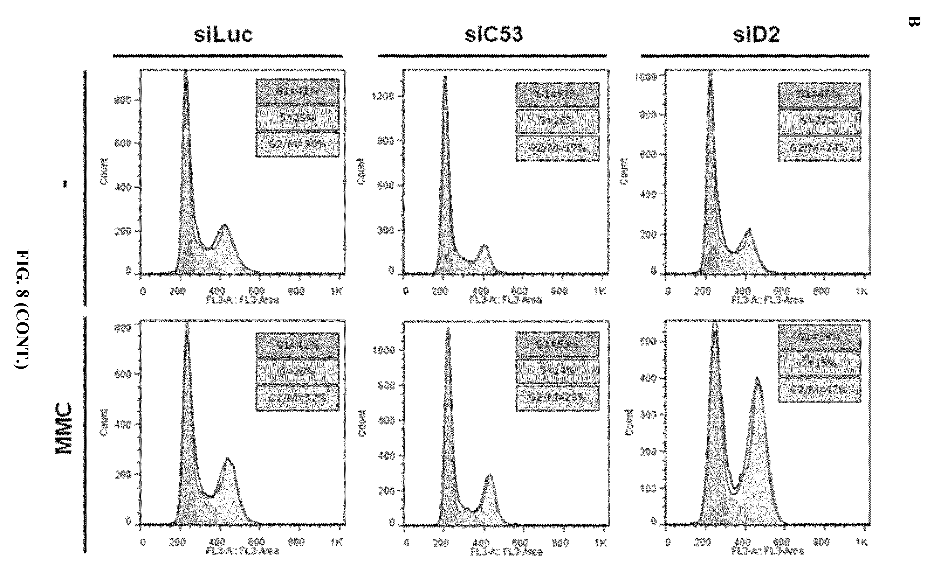

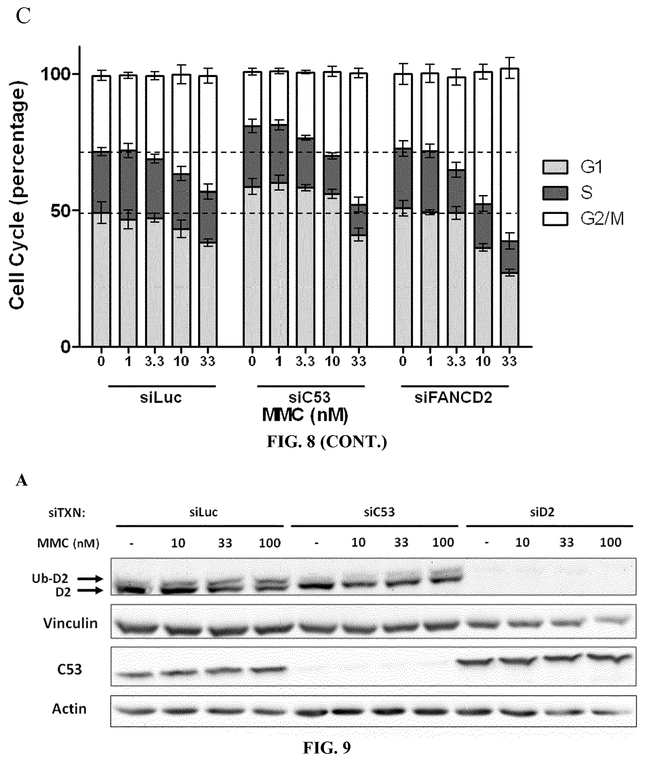

**Figure 8****. Effect of C53 inhibition on cell cycle kinetics.** A) Cell population doubling from siLuc, siC53 or siFANCD2 transfected cells counted in Figure 2C. Graph shows mean ± SEM of 5 independent experiments with no differences in proliferation between siLuc and siC53 transfected cells. B) Representative cell cycle analysis of cells counted in Figure 2C, showing plots from untreated and 10 nM MMC treated cells. C) Cell cycle data from B was averaged and plotted from 4 independent experiments. Bars show mean ± SEM. **p<0.01.

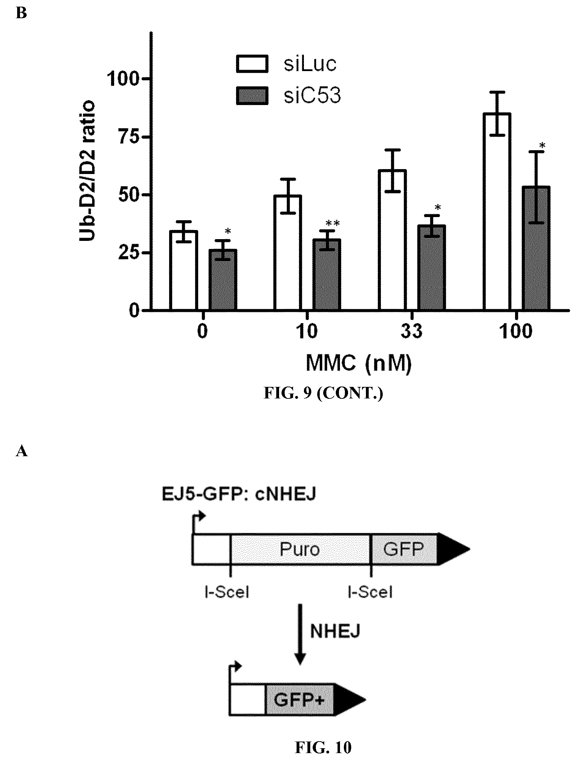

**Figure 9****. Effect of C53 inhibition on FANCD2 ubiquitination.** A) U2OS cells counted in Figure 2C were lysed and western blot was performed to check FA pathway activation by FANCD2 monoubiquitination. B) Graph plot of ubiquitinated vs non ubiquitinated FANCD2 ratio quantified from Western blots with Image J software. Bar shows mean ± SEM of 5 independent experiments. *p<0.05, **p<0.01.

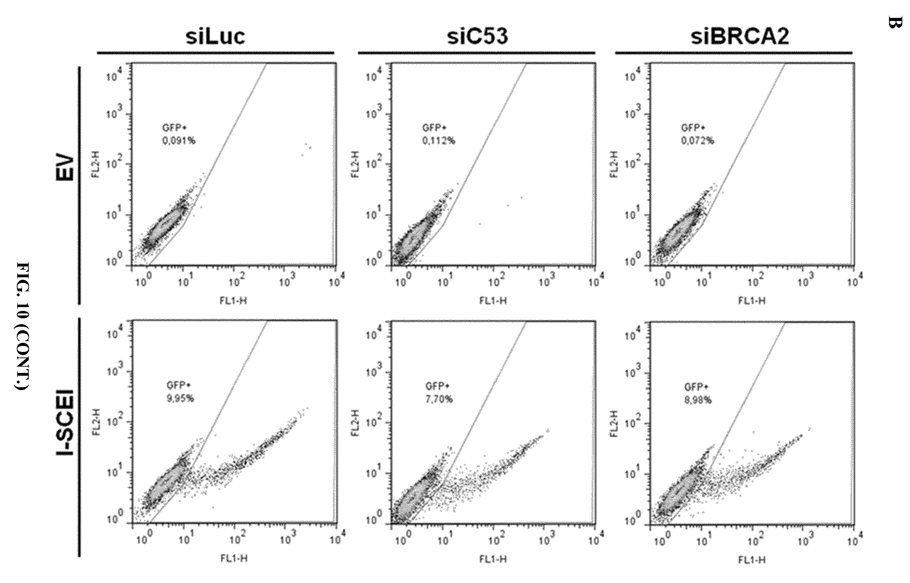

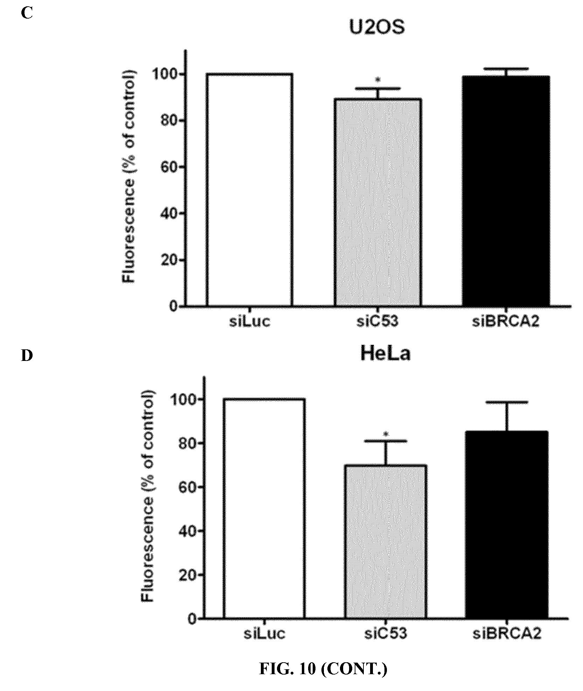

**Figure 10****. C53 contribution in NHEJ repair pathway.** A) Diagram showing the *in vivo* non homologous end joining (NHEJ) repair assay. U2OS or HeLa cells carry inserted in the genome a plasmid with a non-functional GFP coding fragment interrupted by a puromycin resistant coding sequence, flanked with two I-SceI restriction sites. When a plasmid expressing I-SceI endonuclease is transfected, two specific DSBs are generated releasing puromycin coding fragment, and a full, functional GFP coding sequence is formed after DSBs fusion by NHEJ. B) EJ5-GFP stable HeLa cells were transfected with siLuc, siC53 or siBRCA2. After 24h cells were transfected with GFP, I-SceI expressing plasmids or empty vector and 24-48h later fluorescence was analysed by flow cytometry. C-D) Averaged graph from at least three independent experiments as in B were performed in U2OS (C) or Hela (D) cells. GFP transfected cells were used to normalize transfection efficiency. Bars show mean ± SEM. *p<0.05.

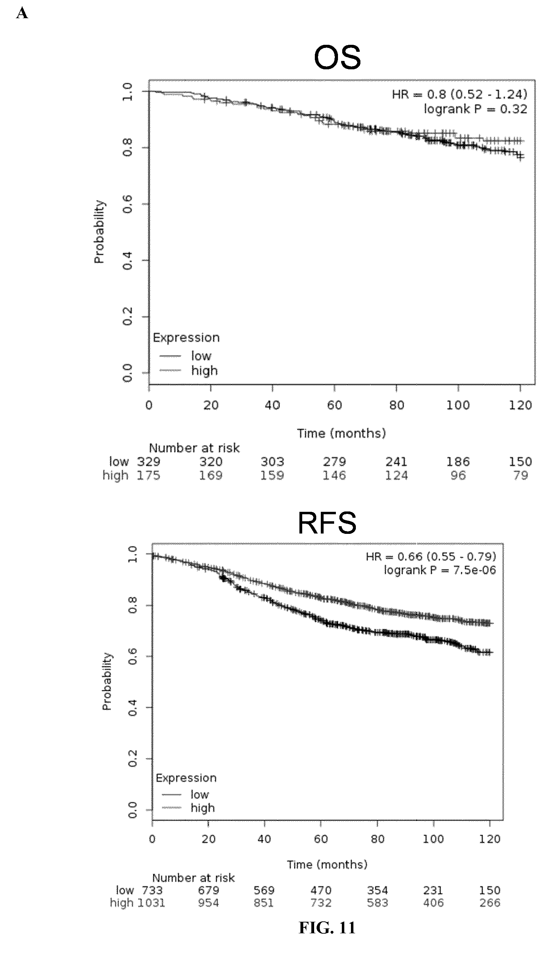

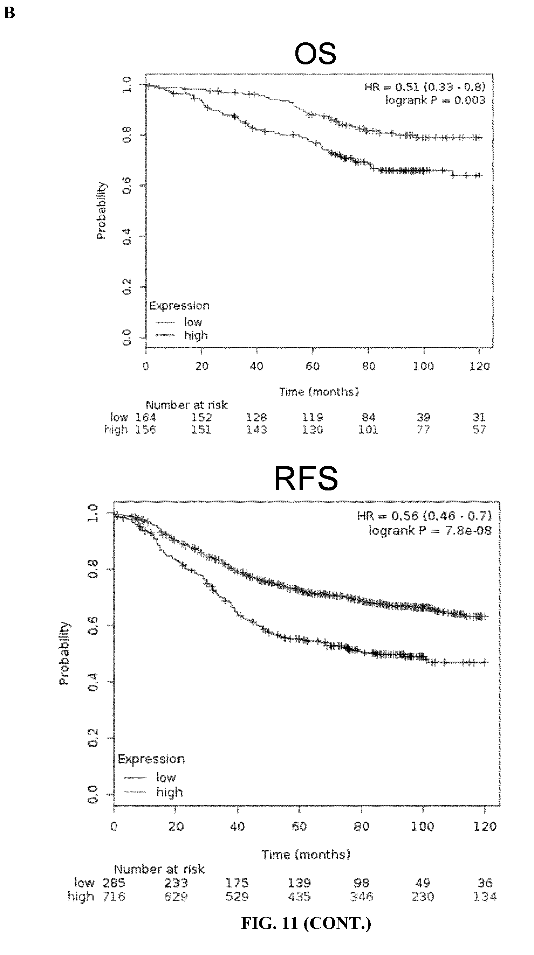

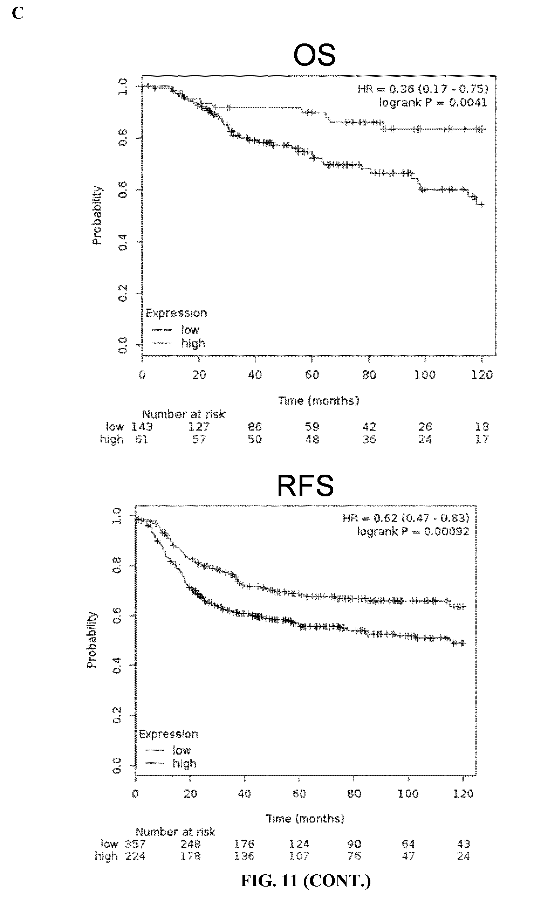

**Figure 11****. Low C53 expression correlation in survival curves for different breast cancer subsets.** Datasets were divided into luminal A (A), luminal B (B) and basal (C).

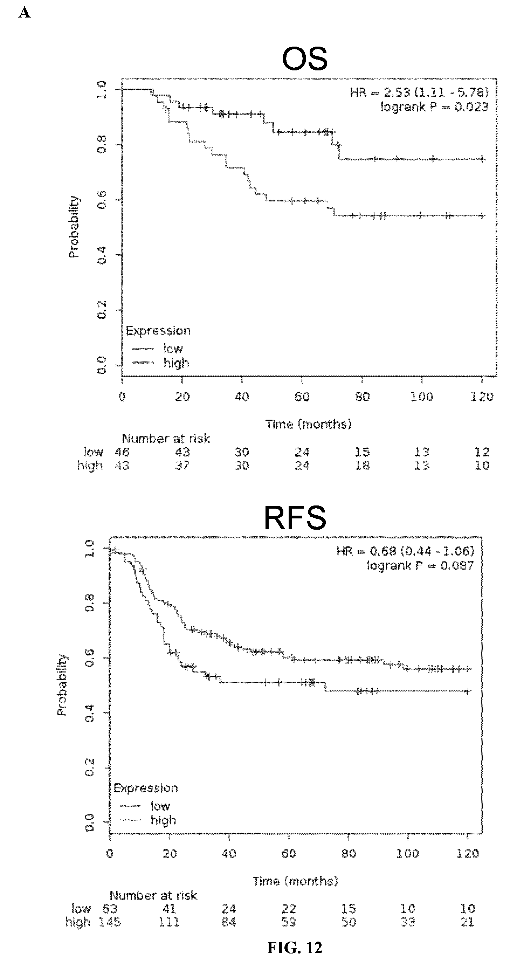

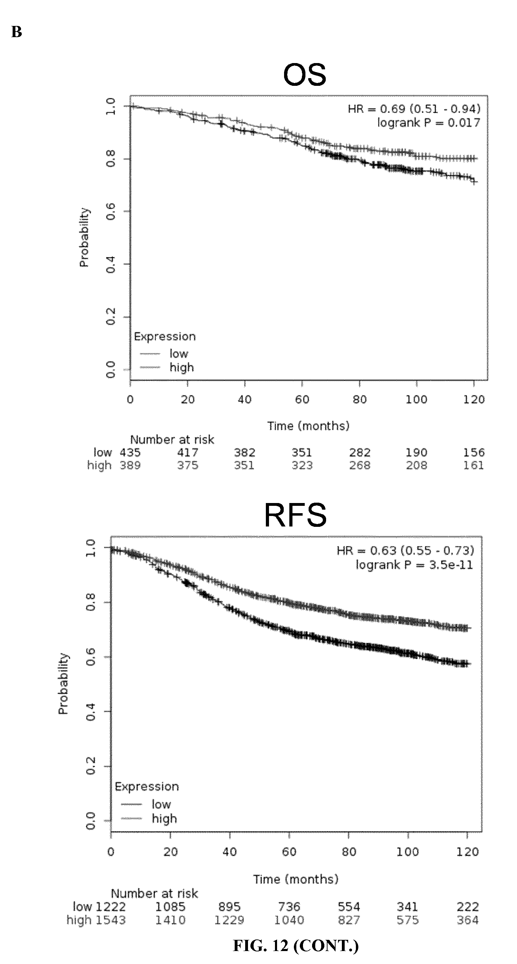

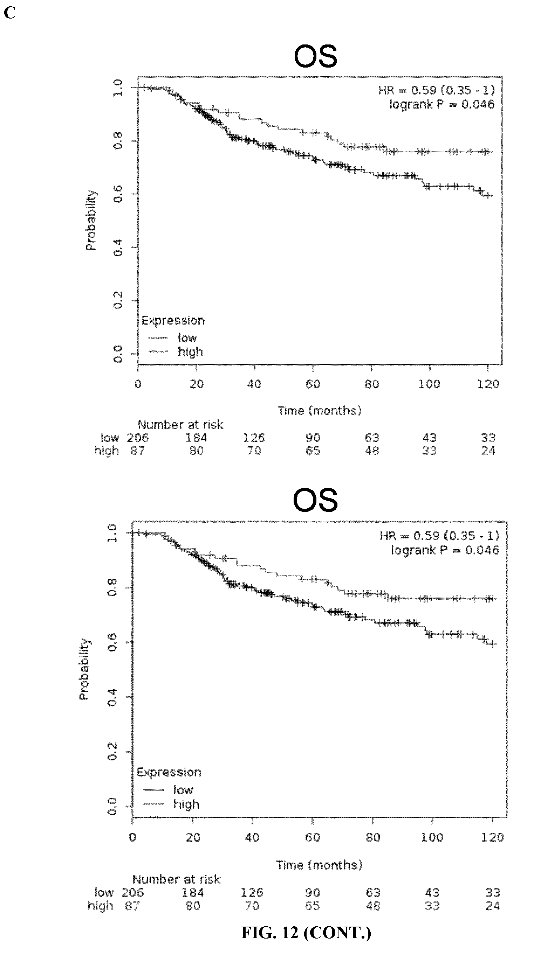

**Figure 12****. Low C53 expression correlation in survival curves for different breast cancer subsets.** Datasets divided in HER2+ (A), ER + (B) and ER - (C).

### DETAILED DESCRIPTION OF THE INVENTION

### Method for determining the prognosis.

In a first aspect, the invention relates to an *in vitro* method for determining the prognosis of a patient suffering from cancer, wherein the cancer is not hepatocarcinoma, hereinafter first method of the invention, comprising the steps of:
(a) determining the expression level of Cyclin-Dependent Kinase 5 Regulatory Subunit Associated Protein 3 (CDK5RAP3) gene in a sample from said patient, and
(b) comparing said expression level with a reference value,
wherein a decreased expression level of CDK5RAP3 gene compared to the reference value is indicative of a poor prognosis or
wherein an increased expression level of CDK5RAP3 gene or a lack of change compared to the reference value is indicative of a good prognosis.

The term "method for determining the prognosis" or "prognostic method", as used herein, refers to the determination of the likelihood that a cancer patient will have a particular clinical outcome, whether positive (good prognosis) or negative (bad prognosis). In the present invention "clinical outcome" is understood as the expected course of a disease. It denotes the doctor's prediction of how a subject's disease will progress, and whether there is chance of recovery, disability or mortality. The prognostic methods of the present invention can be used clinically to make treatment decisions by choosing the most appropriate treatment modalities for any particular patient. As will be understood by those skilled in the art, the prognosis of a cancer, although preferred to be, need not be correct for 100% of the subjects to be diagnosed or evaluated. The term, however, requires that a statistically significant portion of subjects can be identified as suffering cancer. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

The prognosis can be characterized by any parameter which is widely accepted predicting overall survival (OS), progression free survival (PFS), relapse free survival (RFS), long-term survival or disease-specific survival rate.

In a particular embodiment, the prognosis is characterized by predicting overall survival (OS) and/or progression free survival (PFS) and/or relapse free survival (RFS).

The term "overall survival" or "OS", as used herein, refers to the length of time from either the date of diagnosis or the start of treatment for a disease that patients diagnosed with the disease are still alive.

The term "progression free survival" or "PFS", as used herein, refers to the length of time during and after the treatment of a disease that a patient lives with the disease but it does not get worse.

The term "relapse free survival" or "RFS" or "disease-free survival" or "DFS", as used herein, refers to the length of time after primary treatment for a disease that the patient survives without any signs or symptoms of that cancer.

The term "long-term survival", as used herein, refers to survival for a particular period of time. In a particular embodiment, the period of long-term survival is at least 1 year, at least 2 years, at least 3 years, at least 4 years, at least 5 years, at least 6 years, at least 7 years, at least 8 years, at least 9 years, at least 10 years or more.

The term "disease-specific survival rate", as used herein, refers to the percentage of patients who have not died from a specific disease in a period of time beginning at the time of diagnosis or at the start of treatment and ending at the time of death. Patients who die from causes other than the disease are not counted in this measurement.

The term "patient" or "subject", as used herein, refers to any animal, preferably a mammal and includes, but is not limited to, domestic and farm animals, primates and humans, for example, human beings, non-human primates, cows, horses, pigs, sheep, goats, dogs, cats, or rodents. In a preferred embodiment, the subject is a human being of any age or race. In a particular embodiment, the subject suffers from cancer.

The term "cancer", as used herein, refers to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. The term cancer according to the prognostic method of the invention includes any cancer excepting hepatocarcioma, for example: lung cancer, sarcoma, malignant melanoma, pleural mesothelioma, bladder carcinoma, prostate cancer, pancreas carcinoma, gastric carcinoma, ovarian cancer, breast cancer, liver cancer other than hepatocarcinoma, colorectal cancer, kidney cancer, esophageal cancer, suprarenal cancer, parotid gland cancer, head and neck carcinoma, cervix cancer, endometrial cancer, mesothelioma, multiple myeloma, leukaemia, and lymphoma.

The term "hepatocarcinoma", "hepatocellular carcinoma" or "HCC", as used herein, refers to a type of liver cancer that may develop from pre-exisiting dysplastic foci or nodule. HCC arising in cirrhosis is usually preceded by the appearance of non-malignant lesions. According to gross and microscopic features, HCC is differentiated into early HCC: a vaguely nodular lesion with indistinct margins with a well differentiated histology with a few portal tracts detectable within; and progressed HCC: a distinctly nodular lesion with well to moderately differentiated histology in which malignancy is recognized at first glance and no portal tracts detectable within.

In a particular embodiment, the cancer is a breast cancer. The term "breast cancer", as used herein, relates to any malignant proliferative disorder of breast cells, most commonly from the inner lining of milk ducts or the lobules that supply the ducts with milk. Cancers originating from ducts are known as ductal carcinomas, while those originating from lobules are known as lobular carcinomas.

The term "breast cancer" includes all the subtypes of breast cancer classified by the presence or absence of expression of the following receptors:
- Estrogen receptor (ER), which is a member of the nuclear hormone family of intracellular receptors which is activated by estrogen (17-β-estradiol).
- Progesterone receptor (PR), which is member 3 of the nuclear receptor subfamily 3, group C and is activated by the steroid hormone progesterone.
- Human epidermal growth factor receptor 2 (HER2 or HER2/neu), which is a member of the human epidermal growth factor receptor family and is activated by human epidermal growth factor (EGF).

The term "breast cancer" also includes any type of molecular subtypes of breast cancer, like the following:
- Luminal A: ER-positive and/or HER2-negative and with low Ki67.
- Luminal B: ER-positive and/or PR-positive, HER2-positive or HER2-negative with high Ki67.
- Triple negative or basal-like: ER-negative, PR-negative and HER2-negative.
- HER2 type: ER-negative, PR-negative and HER2-postive.

In a more particular embodiment, the cancer is a breast cancer selected from the group consisting of basal-like, luminal A, luminal B, ER+ and ER- breast cancer. In another particular embodiment, the cancer is not a HER2-positive breast cancer.

Methods for diagnosing each molecular subtype of cancer are well known by the person skilled in the art, for example by immunohistochemistry or in situ hybridization.

In a particular embodiment, the cancer is ovarian cancer.

The term "ovarian cancer" or "ovarian, fallopian tube and primary peritoneal cancer", as used herein, refers to a group of tumours that originate in the ovaries, fallopian tubes and peritoneum close to the ovaries and fallopian tubes and includes, without limitation, the following:
- Epithelial tumors: arise from the epithelium covering the ovaries. Ovarian epithelial tumors include the following subtypes:
   - High grade-serous carcinoma
   - Clear cell carcinoma
   - Endometrioid carcinoma
   - Mucinous carcinoma
   - Low-grade serous carcinoma
   - Brenner tumors
   - Undifferentiated tumors
   - Transitional cell tumors
   - Borderline ovarian tumors.
- Germ cell tumors: originate from germ cells which will form the eggs. Germ cell tumors include dysgerminomas and nondisgerminomatous types (embryonal carcinoma, immature teratoma, choriocarcinoma, polyembryoma and mixed germ cell tumors).
- Sex cord-stromal cell tumors: formed from cells of the sex cord or mesenchyme (early connective tissue) within the embryonic gonad and they may contain gonad-related cells (e.g., granulosa cells, Sertoli cells, thecal cells) as well as fibroblasts. Sex cord-stromal cell tumors include granulosa stromal cell tumors and Sertoli- or Sertoli-Leydig cell tumors.

In a particular embodiment, the ovarian cancer is a serous ovarian cancer. The term "serous ovarian cancer", as used herein, refers to an epithelial carcinoma that arises from the lining of a cavity that produces a serum-like fluid. In a more particular embodiment, the serous ovarian cancer is a high-grade serous ovarian cancer. The term "high-grade serous ovarian cancer", as used herein, refers to a serous ovarian cancer characterized by anaplasia and nuclear atypia.

The prognostic method of the invention comprises a first step of determining the expression level of Cyclin-Dependent Kinase 5 Regulatory Subunit Associated Protein 3 (CDK5RAP3) gene in a sample from said patient.

The term "Cyclin-Dependent Kinase 5 Regulatory Subunit Associated Protein 3 gene" or "CDK5RAP3 gene" or "C53 gene", or "LZAP gene" or "IC53 gene" as used herein, refers to a gene encoding the Cyclin-Dependent Kinase 5 Regulatory Subunit Associated Protein 3. The human CDK5RAP3 gene is assigned the Gene ID 80279 (NCBI GenBank, 29 march 2016 update). The term "CDK5RAP3 gene" includes any of the transcript variants that have been described for this gene. The protein encoded by the CDK5RAP3 gene has the amino acid sequence defined under accession number Q96JB5 in UniProtKB/Swiss-Prot (version 132 of the entry as of 16 March 2016 and version 2 of the sequence as 16 June 2003).

The term "expression level", as used herein, refers to a measurable quantity of a gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene.

In a particular embodiment, the expression level of CDK5RAP3 gene is determined by means of the quantification of the level of mRNA encoded by said gene or the quantification of the level of the protein encoded by said gene

The level of a messenger RNA can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA is measured by quantitative or semi-quantitative RT-PCR or by real-time quantitative or semi-quantitative RT-PCR.

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays. Preferably, the quantity of protein is measured by western blot.

Variants of the protein can also be used for measuring the expression levels of the CDK5RAP3 gene in order to put into practice the first method of the invention. Variants of the protein may be: (i) one in which one or more of the amino acid residues are substituted with a conserved or non-conserved amino acid residue (preferably a conserved amino acid residue) and such substituted amino acid residue may or may not be one encoded by the genetic code; (ii) one in which there are one or more modified amino acid residues, e.g., residues that are modified by the attachment of substituent groups; (iii) one in which the protein is an alternative splice variant of the proteins of the present invention and/or; (iv) fragments of the proteins. The fragments include proteins generated via proteolytic cleavage (including multi-site proteolysis) of an original sequence. Variants are deemed to be within the scope of those skilled in the art from the teaching herein.

Variants according to the present invention include amino acid sequences that are at least 60%, 70%, 80%, 90%, 95% or 96% similar or identical to the original amino acid sequence. As known in the art the "similarity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Additionally, the proteins may include unnatural amino acids formed by post-translational modification or by introducing unnatural amino acids during translation.

In a particular embodiment said protein variant is a mammal variant, preferably a human variant, more preferably with at least 60%, 70%, 80%, 90%, 95% or 96% similarity or identity to the original amino acid sequence.

The term "sample", as used herein, refers to biological material isolated from a subject. The biological sample contains any biological material suitable for detecting RNA or protein levels. In a particular embodiment, the sample comprises genetic material, e.g., DNA, genomic DNA (gDNA), complementary DNA (cDNA), RNA, heterogeneous nuclear RNA (hnRNA), mRNA, etc., from the subject under study. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. In a particular embodiment, the sample is a tumor sample, i.e., a sample comprising tumor cells.

The second step of the prognostic method of the invention comprises comparing the expression levels of CDK5RAP3 gene with a reference value.

The term "reference value", as used herein, refers to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, or on a large number of samples, such as from population of subjects of the chronological age matched group, or based on a pool of samples including or excluding the sample to be tested.

In a particular embodiment, the reference value according to the prognostic method of the invention can be obtained from one or more subjects who do not suffer from cancer (i.e., control subjects). In another particular embodiment, the reference value is obtained from one or more subjects who suffer the same type of cancer but have CDK5RAP3 expression levels similar to patients who do not suffer cancer. CDK5RAP3 expression levels are considered similar if they are less than 10%, less than 8%, less than 6%, less than 4%, less than 2%, less than 1%, less than 0.5%, less than 0.1%, less than 0.05%, less than 0.01%, less than 0.001%, higher or lower than CDK5RAP3 expression levels in subjects who do not suffer from cancer.

In a particular embodiment, the reference value according to the prognostic method of the invention is obtained from several subjects who suffer from the same type of cancer than the subject.

When the sample is a tumor sample, the reference value is obtained from a tumor sample from subjects who suffer from the same type of cancer than the subject.

In a more particular embodiment, the reference value can be the median of the level of expression of CDK5RAP3 in several subjects who suffer from the same type of cancer than the patient.

In another more particular embodiment, the reference value according to the prognostic method of the invention is the median of the levels of expression of CDK5RAP3 in several subjects who suffer from the same type of cancer normalized to the expression levels of a housekeeping gene. In this particular embodiment, the prognostic method further comprises normalizing the level of expression of CDK5RAP3 in a sample from the patient to the level of expression of a housekeeping gene in said sample.

The term "housekeeping gene", as used herein, refers to a gene which is generally ubiquitously expressed in all tissues. These genes encode proteins that provide the basic, essential functions that all cells need to survive. Housekeeping genes are usually expressed at the same level in all cells and tissues, but with some variances, especially during cell growth and organism development. Commonly used housekeeping genes include, without limitation, GAPDH (glyceraldehyde-3-phosphate dehydrogenase), beta-actin gene, the tubulin gene, the vinculin gene, genes coding for the 18S or 28S rRNA subunits of the ribosome. An exemplary listing of housekeeping genes can be found, for example, in Trends in Genetics, 19, 362-365 (2003).

The term "normalization", as used herein, means conversion of the quantitative numerical values into numerical values which can be compared with gene expression amounts obtained by other gene expression analyses. Typically, normalization is carried out by using the gene expression levels of a housekeeping gene being steadily expressed as an index for normalization of gene expression amounts.

According to the prognostic method of the invention, the level or the expression level of CDK5RAP3 is considered "decreased" when the expression level of CDK5RAP3 in a sample is lower than its reference value. The expression level of CDK5RAP3 is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the prognostic method of the invention, the expression level of CDK5RAP3 is considered "increased" when the expression level of CDK5RAP3 in a sample is higher than its reference value. The expression level of CDK5RAP3 is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

According to the prognostic method of the invention, there is a lack of change of the expression level of CDK5RAP3 gene compared to a reference value when the expression level is similar to the reference value, that is, the expression level of CDK5RAP3 gene is less than 10%, less than 8%, less than 6%, less than 4%, less than 2%, less than 1%, less than 0.5%, less than 0.1%, less than 0.05%, less than 0.01%, less than 0.001%, higher or lower than its reference value.

According to the prognostic method of the invention, a decreased expression level of CDK5RAP3 gene compared to the reference value is indicative of a poor prognosis and an increased expression level of CDK5RAP3 gene or a lack of change compared to the reference value is indicative of a good prognosis.

The term "good prognosis", as used herein, refers to an outcome which would be regarded positive for the patient. In a particular embodiment, the good prognosis refers to the likelihood of long-term survival, overall survival, disease-specific survival, progression-free survival or disease-free survival. In a particular embodiment, a good prognosis includes stabilization of the disease.

The term "poor prognosis", as used herein, means an outcome which would be regarded negative for the patient. In a particular embodiment, a bad prognosis includes a prediction of relapse, disease progression or mortality.

### Method for predicting the clinical response

In a second aspect, the invention relates to an *in vitro* method for predicting the clinical response of a patient suffering from cancer to a treatment with a DNA damaging agent, hereinafter second method of the invention, comprising the steps of:
(a) determining the expression level of CDK5RAP3 gene in a sample from said patient and
(b) comparing said expression level with a reference value,
wherein a decreased expression level of CDK5RAP3 gene compared to the reference value is indicative of a poor clinical response of the patient to said treatment.

The term "predicting the clinical response", as used herein, refers to the likelihood that a patient will have a particular clinical outcome, whether positive or negative. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen. The prediction may also include prognostic factors. As it will be understood by those skilled in the art, the prediction, although preferred to be, need not be correct for 100% of the patients to be evaluated. The term, however, requires that a statistically significant portion of subjects can be identified as having an increased probability of having a given clinical response.

The term "poor response", as used herein, refers to not obtaining beneficial or desired clinical results which can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable.

In a particular embodiment, an increased expression level of CDK5RAP3 gene or a lack of change compared to the reference value is indicative of a good clinical response of the patient to said treatment. The terms "increased expression level" and "lack of change" have been previously defined in connection with the first method of the invention. The term "good clinical response", as used herein, refers to obtaining beneficial or desired clinical results which can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable.

The term "treatment", as used herein, refers to any process, action, application, therapy, or the like, wherein a subject (or patient), including a human being, is provided medical aid with the object of improving the subject's condition, directly or indirectly, or slowing the progression of a condition or disorder in the subject, or ameliorating at least one symptom of the disease or disorder under treatment.

The term "DNA damaging agent", as used herein, refers to substances or treatments which induce DNA damage or which inhibit DNA damage response.

DNA damaging agents includes the following:
- Interstrand-crosslink inducers: agents that covalently bind to two nucleotide residues of different DNA strands. Illustrative, non-limitative examples of interstrand-crosslink inducers are nitrogen mustards and derivatives (including cyclophosphamide, melphalan, chlorambucil, ifosfamide, mechlorethamine and uramustine), nitrosoureas (including carmustine, lomustine, semustine and streptozotocin), alkyl sulfonates (including busulfan), ethylenimines (including thiotepa and altretamine), triazenes (including procarbazine, dacarbazine and temozolomide), platinums (including cisplatin, carboplatin, oxaliplatin, satraplatin, picoplatin, nedaplatin and triplatin), mitomycin C and psoralens.
- Double-strand break inducers: agents that induce a break in both DNA strands. Illustrative, non-limitative examples of interstrand-crosslink inducers include ionizing radiation (gamma radiation, X radiation and far ultraviolet radiation), topoisomerase I inhibitors (irinotecan, camptothecin, topotecan) and topoisomerase II inhibitors (doxorubicin, epirubicin, etoposide, teniposide, mitoxantrone).
- PARP inhibitors: agents that interfere with the ability of PARP to repair DNA single-strand breaks. The term "PARP" or "poly ADP ribose polymerase", as used herein, refers to a family of enzymes involved in DNA repair and programmed cell death (EC number 2.4.2.30). Illustrative, non-limitative examples of PARP inhibitors include iniparib, talazoparib, olaparib, rucapariv, veliparib, CEP 9722, AK 4827, BGB-290 and 3-aminobenzamide.
- DNA intercalating agents: agents capable of binding by insertion between DNA base-pairs. Illustrative, non-limitative examples of DNA intercalating agents include berberine, ethidium bromide, proflavine, daunomycin, doxorubicin, thalidomide, ethidium homodimer, mitonafide, ametantrone; 1,10-phenanthroline; elliptinium; gilvocarcin V; amonafide; diminazene aceturate; DAPI; ferroin; amsacrine; propidium; nogalamycin; acriflavine; 9-amino-6-chloro-2-methoxyacridine; angelicin; 4-((3-bromophenyl)amino)-6,7-dimethoxyquinazoline; 1,1'-(4,4,7,7-tetramethyl -4,7-diazaundecamethylene) bis-4-(3-methyl-2,3-dihydro (benzo-1,3-thiazole) -2-methylidene) quinolinium; 1,1'- ((4,4,7,7-tetramethyl) -4,7-diazaundecamethylene) bis- 4-(3-methyl-2,3-dihydro(benzo-1,3-oxazole) -2-methylidene) quinolinium; NSC 366140 and ditercalinium.
- Alkylating agents: agents that mediate the transfer of an alkyl group from one molecule to DNA. The alkyl group may be transferred as an alkyl carbocation, a free radical, a carbanion or a carbine (or their equivalents). Illustrative, non-limitative examples of alkylating agents include nitrogen mustards, nitrosoureas, ethylenimine derivatives, alkyl sulfonates and triazenes, including, but not limited to, cyclophosphamide (Cytoxan™), busulfan, improsulfan, piposulfan, pipobroman, melphalan (L-sarcolysin), chlorambucil, mechlorethamine or mustine, uramustine or uracil mustard, novembichin, phenesterine, trofosfamide, ifosfamide, carmustine (BCNU), lomustine (CCNU), chlorozotocin, fotemustine, nimustine, ranimnustine, semustine (methyl-CCNU), streptozocin, thiotepa, triethylenemelamine, triethylenethiophosphoramine, procarbazine, altretamine, dacarbazine, mitozolomide and temozolomide.
- Nucleoside analogs: analogues of physiological pyrimidine and purine nucleosides that have cytotoxic activity by being incorporated into and altering the DNA, by interfering with various enzymes involved in synthesis of nucleic acids or by modifying the metabolism of physiological nucleosides. Illustrative, non-limitative examples of nucleoside analogues include purine analogues, like fludarabine and cladribine; pyrimidine analogues, like cytarabine and gemcitarabine, and fluoropyrimidines, like fluorouracile and capecitabine.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C. The term "mitomycin C", as used herein, refers to a compound with the following structure:

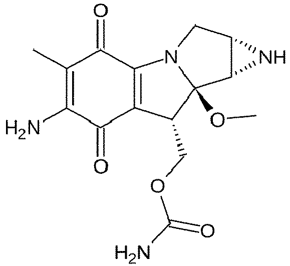

In another particular embodiment, the double-strand break inducer is ionizing radiation. The term "ionizing radiation", as used herein, refers to a radiation that carries enough energy to free electrons from atoms or molecules, thereby ionizing them. The term "ionizing radiation" includes gamma rays, X-rays and the higher ultraviolet part of the electromagnetic spectrum. In an even more particular embodiment, the ionizing radiation is gamma radiation. The term "gamma radiation", as used herein, refers to a high-frequency electromagnetic radiation originated in the atomic nucleus.

In another particular embodiment, the PARP inhibitor is olaparib or veliparib. The term "olaparib", as used herein, refers to a compound with the following structure:

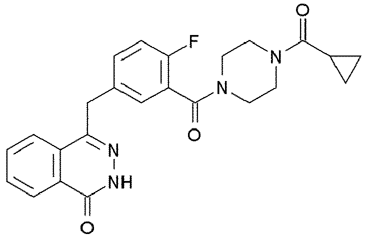

The term "veliparib", as used herein, refers to a compound with the following structure:

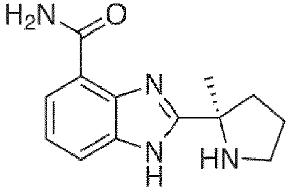

The terms *"in vitro",* "cancer", "patient", "expression level", "CDK5RAP3 gene", "sample", "decreased expression" have been defined in connection with the prognostic method of the invention. All the particular embodiments of these terms defined in connection with the prognostic method of the invention apply to the predictive method of the invention.

In a particular embodiment, the cancer is osteosarcoma. The term "osteosarcoma", as used herein, refers to an aggressive cancerous neoplasm arising from primitive transformed cells of mesenchymal origin that exhibit osteoblastic differentiation and produce malignant osteoid. Osteosarcoma is classified into low-grade and high-grade according to the appearance of the cancer cells. Low-grade osteosarcoma is characterized by cancer cells with normal appearance, with slow growth and high-grade osteosarcoma is characterized by cancer cells with very abnormal appearance, with rapid growth and more likely to spread. Most osteosarcomas are high-grade, but a type known as a parosteal osteosarcoma is usually low-grade. A further subtype (periosteal osteosarcoma) is usually treated as though it was high-grade. In addition, osteosarcoma can be classified according to its localization in the following stages:
- Stage 1A: low-grade osteosarcoma completely contained within the hard coating of the bone;
- Stage 1B: low-grade osteosarcoma extending outside the bone into the soft tissue spaces, which contain nerves and blood vessels;
- Stage 2A: high-grade osteosarcoma completely contained within the hard coating of the bone;
- Stage 2B: high-grade osteosarcoma extending outside the bone into the soft tissue spaces, which contain nerves and blood vessels. Most osteosarcomas are stage 2B;
- Stage 3: low-grade or high-grade osteosarcoma found either within the bone or extending outside the bone, that has metastasized.

In another particular embodiment, the cancer is breast cancer or ovarian cancer. In a more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

### Method for selecting a patient for a therapy

In a third aspect, the invention relates to an *in vitro* method for selecting a patient suffering from cancer for a therapy with a DNA damaging agent, hereinafter third method of the invention, comprising the steps of:
(a) determining the expression level of CDK5RAP3 gene in a sample from said patient and
(b) comparing said expression level with a reference value,
wherein the patient is selected for said therapy if the expression level of CDK5RAP3 gene is not decreased compared to the reference value.

The expression "selecting a patient for a therapy", as used herein, relates to the identification of a patient for a therapy designed to cure a disease or palliate the symptoms associated with one or more diseases or conditions. As will be understood by those skilled in the art, the selection of a patient, although preferred to be, need not be adequate for 100% of the subjects selected according to the third method of the invention. The term, however, requires that a statistically significant portion of subjects were correctly selected. Whether the selection of a patient in a population of subjects is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.01, 0.05, 0.005, 0.001 or lower.

The terms *"in vitro",* "cancer", "patient", "expression level", "CDK5RAP3 gene", "sample", "decreased expression", "DNA damaging agent" have been defined in connection with the first and second methods of the invention. All the particular embodiments of these terms defined in connection with the prognostic method of the invention or the second method of the invention apply to the third method of the invention.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C.

In another particular embodiment, the double-strand break inducer is ionizing radiation. In an even more particular embodiment, the ionizing radiation is gamma radiation.

In another particular embodiment, the PARP inhibitor is olaparib or veliparib.

In a particular embodiment, the cancer is osteosarcoma.

In a particular embodiment, the cancer is breast cancer or ovarian cancer. In a more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

### Method for selecting a therapy for a patient suffering from cancer

In a fourth aspect, the invention relates to an *in vitro* method for selecting a therapy for a patient suffering from cancer, hereinafter fourth method of the invention, comprising the steps of
(a) determining the expression level of CDK5RAP3 gene in a sample from said patient and
(b) comparing said expression level with a reference value,
wherein if the expression level of CDK5RAP3 gene is not decreased compared to the reference value, a therapy with a DNA damaging agent is selected and
wherein if the expression level of CDK5RAP3 gene is decreased compared to the reference value, an alternative therapy is selected.

The term "selecting a therapy for a patient", as used herein, relates to the identification of a therapy or treatment to cure a disease or palliate the symptoms of the disease in a particular patient.

The term "alternative therapy", as used herein, means a therapy which inhibits or suppresses the growth and proliferation of cancer cells which is not a DNA damaging agent. Illustrative, non-limitative examples of alternative therapies include compounds that destroy cancer cells or interfere with cell division, compounds that block certain hormones involved in cancer, compounds that inhibit or prevent the growth of new blood vessels (e.g. angiogenesis inhibitors) and compounds with anticancer properties (e.g., taxanes, vinca alkaloids, and plant alkaloids).

In a particular embodiment, the alternative therapy is selected from the group consisting of an inhibitor of Rad52, an inhibitor of XPF, a Weel inhibitor, a Chk1 inhibitor, a Chk2 inhibitor and a MDM2 inhibitor.

The term "DNA repair protein RAD52 homolog" or "Rad52", as used herein, refers to a protein involved in DNA double-strand break repair and homologous recombination. In humans it is encoded by the RAD52 gene, identified by the gene ID 5893 (NCBI GenBank, 3 April 2016 update). Human Rad52 is identified by accession number P43351 in UniProtKB/Swiss-Prot (entry version 147 as of 16 March 2016 and sequence version 1 as of 1 November 1995).

The term "DNA repair endonuclease XPF" or "ERCC4", as used herein, refers to a protein that forms together with ERCC1 the ERCC1-XPF enzyme complex that participates in DNA repair and recombination. In humans it is encoded by the *ERCC4* gene, identified by the gene ID 2072 (NCBI GenBank, 3 April 2016 update). Human DNA repair endonuclease XPF is identified by the accession number Q92889 in UniProtKB/Swiss-Prot (entry version 169 as of 16 March 2016 and sequence version 3 as of 5 May 2009).

The term "Weel-like protein kinase" or "Weel kinase", as used herein, refers to a serine/threonine protein kinase which inhibits Cdkl by phosphorylating it on tyrosine 15 and threonine 14. In humans it is encoded by the WEE1 gene, identified by the gene ID 7465 (NCBI GenBank, 3 April 2016 update). Human Weel-like protein kinase is identified by the accession number P30291 in UniProtKB/Swiss-Prot (entry version 175 as of 16 March 2016 and sequence version 2 as of 1 February 1996).

The term "serine/threonine-protein kinase Chkl" or "CHK1 checkpoint homolog" or "cell cycle checkpoint kinase" or "checkpoint kinase-l" or "Chkl", refers to a serine/threonine protein kinase which coordinates the DNA damage response and cell cycle checkpoint response. In humans it is encoded by the CHEK1 gene, identified by the gene ID 1111 (NCBI GenBank, 3 April 2016 update). Human serine/threonine-protein kinase Chkl is identified by the accession number 014757 in UniProtKB/Swiss-Prot (entry version 187 as of 16 March 2016 and sequence version 2 as of 11 January 2011).

The term "serine/threonine-protein kinase Chk2" or "CHK2 checkpoint homolog" or "checkpoint kinase-2" or "Chk2", refers to a serine/threonine protein kinase which coordinates the DNA damage response and cell cycle checkpoint response. In humans it is encoded by the CHEK2 gene, identified by the gene ID 11200 (NCBI GenBank, 3 April 2016 update). Human serine/threonine-protein kinase Chk2 is identified by the accession number 096017 in UniProtKB/Swiss-Prot (entry version 191 as of 16 March 2016 and sequence version 1 as of 1 May 1999).

The term "E3 ubiquitin-protein ligase Mdm2" or "mouse double minute 2 homolog" or "oncoprotein Mdm2" or "p53-binding protein Mdm2" or "MDM2", refers to an E3 ubiquitin ligase that recognizes the N-terminal transactivation domain of p53 and inhibits p53 transcriptional activation. In humans it is encoded by the *MDM2* gene, identified by the gene ID 4193 (NCBI GenBank, 3 April 2016 update). Human E3 ubiquitin-protein ligase Mdm2 is identified by the accession number Q00987 in UniProtKB/Swiss-Prot (entry version 209 as of 16 March 2016 and sequence version 1 as of 1 April 1993).

The term "inhibitor", as used herein, refers to a compound inhibiting the activity of a target protein, including, without limitation, antagonists, inhibitory antibodies, compounds which prevent the expression of the gene encoding the protein and expression and compounds which lead to reduced mRNA or protein levels.

Illustrative non-limitative examples of inhibitors of Rad52 are the small compounds described by Huan et al., Nucleic Acids Research 2016, doi:10.1093/nar/gkw087; Sullivan et al., PLoS One 2016, 11: e0147230.

Illustrative non-limitative examples of inhibitors of ERCC4 are the small compounds described by Jordheim et al, Mol Pharmacol 2013, doi: 10.1124/mol.112.082347; McNeil et al, DNA Repair 2015, doi: 10.1016/j.dnarep.2015.04.002.

Illustrative non-limitative examples of inhibitors of Weel kinase are small molecules like 2-allyl-1- (6-(2-hydroxypropan-2-yl) pyridin-2-yl) -6-((4-(4-methylpiperazin-1-yl) phenyl) amino) -1H-pyrazolo[3,4-d]pyrimidin-3(2H)-one (AZD1775 or MK-1775); 4-(2-Chlorophenyl)-9-hydroxypyrrolo[3,4-c]carbazole-1,3-(2H,6H)-dione (CAS number 622855-37-2); PD 166285 or heparin-cantithrombin II (CAS 212391-63-4); PD 407824 or 9-Hydroxy-4-phenylpyrrolo[3,4-c]carbazole-1,3(2H,6H)-dione (CAS number 622864-54-4); 4-(2-Phenyl)-9-hydroxypyrrolo[3,4-c]carbazole-1,3-(2H,6H)-dione (CAS number 1177150-89-8); 4-(2-Chlorophenyl)-9-hydroxypyrrolo[3,4-c]carbazole-1,3-(2H,6H)-dione (CAS number 622855-37-2).

Illustrative non-limitative examples of inhibitors of Chkl are:
- AZD-7762 or 5-(3-Fluorophenyl)-3-ureidothiophene-2-carboxylic acid N-[(S)-piperidin-3-yl]amide hydrochloride (CAS number 1246094-78-9);
- SCH900776 or MK-8776 or 6-Bromo-3-(1-methyl-1H-pyrazol-4-yl)-5-(3R)-3-piperidinylpyrazolo[1,5-a]pyrimidin-7-amineMK-8776 (CAS number 891494-63-6);
- IC83 or LY2603618 or (S)-1-(5-bromo-4-methyl-2-(morpholin-2-ylmethoxy)phenyl)-3-(5-methylpyrazin-2-yl)urea (CAS number 911222-45-2);
- LY2606368 or (Z)-5-((5-(2-(3-aminopropoxy)-6-methoxyphenyl)-1H-pyrazol-3(2H)-ylidene)amino)pyrazine-2-carbonitrile (CAS number 1234015-52-1);
- PF-00477736 or (2R)-2-Amino-2-cyclohexyl-N-[2-(1-methyl-1H-pyrazol-4-yl)-6-oxo-5,6-dihydro-1H-[1,2]diazepino[4,5,6-cd]indol-8-yl]-acetamide (CAS number 952021-60-2);
- SAR-020106 (CAS number 1184843-57-9);
- CCT-244747 or (R)-3-((1-(dimethylamino)propan-2-yl)oxy)-5-((4-methoxy-5-(1-methyl-1H-pyrazol-4-yl)pyridin-2-yl)amino)pyrazine-2-carbonitrile (CAS number 1404095-34-6);
- PD 407824 or 9-Hydroxy-4-phenylpyrrolo[3,4-c]carbazole-1,3(2H,6H)-dione (CAS number 622864-54-4);
- 4-(2-Phenyl)-9-hydroxypyrrolo[3,4-c]carbazole-1,3-(2H,6H)-dione (CAS number 1177150-89-8).

Illustrative non-limitative examples of inhibitors of Chk2 are:
- AZD-7762 or 5-(3-Fluorophenyl)-3-ureidothiophene-2-carboxylic acid N-[(S)-piperidin-3-yl]amide hydrochloride (CAS number 1246094-78-9);
- LY2606368 or (Z)-5-((5-(2-(3-aminopropoxy)-6-methoxyphenyl)-1H-pyrazol-3(2H)-ylidene)amino)pyrazine-2-carbonitrile (CAS number 1234015-52-1);
- PF-00477736 or (2R)-2-Amino-2-cyclohexyl-N-[2-(1-methyl-1H-pyrazol-4-yl)-6-oxo-5,6-dihydro-1H-[1,2]diazepino[4,5,6-cd]indol-8-yl]-acetamide (CAS number 952021-60-2);
- CCT-244747 or (R)-3-((1-(dimethylamino)propan-2-yl)oxy)-5-((4-methoxy-5-(1-methyl-1H-pyrazol-4-yl)pyridin-2-yl)amino)pyrazine-2-carbonitrile (CAS number 1404095-34-6);

Illustrative non-limitative examples of inhibitors of Mdm2 are:
- Nutlin-3 o (±)-4-[4,5-bis(4-chlorophenyl)-2-(4-methoxy-2-propan-2-yloxyphenyl)-4, 5-dihydroimidazole-1-carbonyl]piperazin-2-one (CAS number 548472-68-0);
- HLI 373 or 5-[[3-Dimethylamino)propyl]amino]-3,10-dimethylpyrimido[4,5-b]quinoline-2,4(3H,10H)-dione dihydrochloride (CAS number 502137-98-6);
- JNJ 26854165 or N1-(2-(1H-Indol-3-yl)ethyl)-N4-(pyridin-4-yl)benzene-1,4-diamine (CAS number881202-45-5);
- (-)-Nutlin-3 or (-)-4-(4,5-Bis(4-chlorophenyl)-2-(2-isopropoxy-4-methoxyphenyl)-4,5-dihydro-1H-imidazole-1-carbonyl)piperazin-2-one (CAS number 675576-98-4);
- trans-4-Iodo-4'-boranyl-chalcone or MDM2 Antagonist IV (CAS number 562823-84-1)
- NSC 66811 or 7-(anilino(phenyl)methyl)-2-methyl-8-quinolinol (CAS number 6964-62-1);
- R5C3 or 5-Methyl-N-(2-phenoxybenzoyl)-1-[(phenylmethoxy)carbonyl]-D/L-tryptophan (CAS number753504-14-2).

The terms *"in vitro",* "cancer", "patient", "expression level", "CDK5RAP3 gene", "sample", "decreased expression", "DNA damaging agent" have been defined in connection with the first, second and third methods of the invention. All the particular embodiments of these terms defined in connection with the first, second and third methods of the invention apply to the fourth method of the invention.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C.

In another particular embodiment, the double-strand break inducer is ionizing radiation. In an even more particular embodiment, the ionizing radiation is gamma radiation.

In another particular embodiment, the PARP inhibitor is olaparib or veliparib.

In a particular embodiment, the cancer is osteosarcoma.

In a particular embodiment, the cancer is breast cancer or ovarian cancer. In a more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

### Therapeutic method

In a fifth aspect, the invention relates to a method for the treatment of a patient suffering from cancer, hereinafter fifth method of the invention, comprising administering a therapy with a DNA damaging agent if the expression level of CDK5RAP3 gene in a sample from said patient is not decreased compared to a reference value.

The terms "cancer", "patient", "expression level", "CDK5RAP3 gene", "sample", "decreased expression", "DNA damaging agent" have been defined in connection with the first, second and third methods of the invention. All the particular embodiments of these terms defined in connection with the first, second and third methods of the invention apply to the fifth method of the invention.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C.

In another particular embodiment, the double-strand break inducer is ionizing radiation. In an even more particular embodiment, the ionizing radiation is gamma radiation.

In another particular embodiment, the PARP inhibitor is olaparib or veliparib.

In a particular embodiment, the cancer is osteosarcoma.

In a particular embodiment, the cancer is breast cancer or ovarian cancer. In a more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

### Uses of the invention

In a sixth aspect, the invention relates to the use of a reagent capable of determining the expression level of CDK5RAP3 gene in a sample from a subject suffering from cancer for determining the prognosis of said patient, wherein the cancer is not hepatocarcinoma, or for predicting the clinical response of a patient suffering from cancer to a treatment with a DNA damaging agent, or for selecting a patient suffering from cancer for a therapy with a DNA damaging agent, or for selecting a therapy for a patient suffering from cancer.

The term "reagent capable of determining the expression level of CDK5RAP3 gene", as used herein, refers to any reagent suitable for detecting the gene product produced by CDK5RAP3 gene, wherein the gene product can be a transcriptional product (mRNA) or a translational product (protein encoded by the CDK5RAP3 gene). In a particular embodiment, the reagent capable of determining the expression level of CDK5RAP3 is a nucleic acid capable of hybridizing specifically with the CDK5RAP3 mRNA. Nucleic acids capable of hybridizing specifically with the CDK5RAP3 mRNA are, for example, one or more pairs of primer oligonucleotides for the specific amplification of fragments of the mRNA (or their correspondent cDNA) of CDK5RAP3 and/or one or more probes for the identification of CDK5RAP3 mRNA. As the skilled person understands, the oligonucleotide primers and probes can be used in all techniques of gene expression profiling (RT-PCR, SAGE, TaqMan, Real Time-PCR, FISH, NASBA, etc).

In another particular embodiment, the reagent capable of determining the expression level of CDK5RAP3 is an antibody that specifically binds to the protein encoded by CDK5RAP3 gene. Antibodies, or fragments thereof, capable of specifically binding to CDK5RAP3 protein or to variants thereof are, for example, monoclonal and polyclonal antibodies, antibody fragments, Fv, Fab, Fab' and F(ab')2, ScFv, diabodies, triabodies, tetrabodies and humanised antibodies. These antibodies can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc.

The terms "cancer", "patient", "expression level", "CDK5RAP3 gene", "sample", "determining the prognosis", "predicting the clinical response", "selecting a patient", "selecting a therapy", "DNA damaging agent" have been defined in connection with the first, second, third and fourth methods of the invention. All the particular embodiments of these terms defined in connection with the methods of the invention apply to the sixth aspect of the invention.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C.

In another particular embodiment, the double-strand break inducer is ionizing radiation. In an even more particular embodiment, the ionizing radiation is gamma radiation.

In another particular embodiment, the PARP inhibitor is olaparib or veliparib.

In a particular embodiment, the cancer is osteosarcoma.

In a particular embodiment, the cancer is breast cancer or ovarian cancer. In a more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

### Methods of personalized medicine

In a seventh aspect, the invention relates to a DNA damaging agent-based treatment for use in the treatment of cancer in a patient, wherein a sample of said patient shows increased or equal expression levels of CDK5RAP3 gene compared to a reference value.

Alternatively (or additionally), the invention relates to the DNA damaging agent-based treatment for use according to the seventh aspect, wherein the patient has been previously selected by showing increased or equal expression levels of CDK5RAP3 gene compared to a reference value in a sample from said patient.

Alternatively (or additionally), the invention relates to the DNA damaging agent-based treatment for use according to the seventh aspect, wherein the patient, prior to the treatment, is selected by a method comprising determining the expression level of CDK5RAP3 gene in a sample from said patient.

The terms "cancer", "patient", "expression level", "CDK5RAP3 gene", "sample", "increased expression", "DNA damaging agent", "reference value" have been defined in connection with the first, second and third methods of the invention. All the particular embodiments of these terms defined in connection with the first, second and third methods of the invention apply to the seventh aspect of the invention.

According to the method of the seventh aspect, the level or the expression level of CDK5RAP3 is considered "equal" when the expression level of CDK5RAP3 in a sample is substantially the same than its reference value. The expression level of CDK5RAP3 is considered to be substantially the same than its reference value when it is less than 10%, less than 8%, less than 6%, less than 4%, less than 2%, less than 1%, less than 0,5%, less than 0,1%, less than 0,05%, less than 0,01%, less than 0,001%, higher or lower than its reference value.

In a particular embodiment, the DNA damaging agent is selected from the group consisting of an interstrand-crosslink inducer, a double-strand break inducer and a PARP inhibitor.

In a more particular embodiment, the interstrand-crosslink inducer is mitomycin C.

In another particular embodiment, the double-strand break inducer is ionizing radiation. In an even more particular embodiment, the ionizing radiation is gamma radiation.

In another particular embodiment, the PARP inhibitor is olaparib or veliparib.

In a particular embodiment, the cancer is osteosarcoma.

In a particular embodiment, the cancer is breast cancer or ovarian cancer. In more particular embodiment, the breast cancer is selected from the group consisting of basal-like, luminal A, luminal B, ER+ and ER- breast cancer.

In a particular embodiment, the cancer is not hepatocarcinoma.

In a particular embodiment, the sample is a tumor sample.

In a particular embodiment, the expression level of CDK5RAP3 gene is determined by means of the quantification of the level of mRNA encoded by said gene or the quantification of the level of the protein encoded by said gene.

In a particular embodiment, the cancer is not a HER2-positive breast cancer.

The invention is detailed below by means of the following examples which are merely illustrative and by no means limiting for the scope of the invention.

### EXAMPLES

### Materials and Methods

### Reagents and antibodies

Mitomycin C (MMC, M0503), 5-bromo-2-deoxyuridine (BrdU, B5002) and Hoechst 33258 (B2883) were from Sigma. PARP inhibitor Olaparib (S1060) was from Selleckchem. Propidium iodide (P3566) and RNAse (12091) were from Invitrogen. Colcemid was from Gibco. Antibodies used: mouse monoclonal to C53 (ab57817), rabbit polyclonal to BRCA2 (ab123491), rabbit polyclonal to FANCD2 (ab2187), rabbit polyclonal to actin (ab1801) and mouse monoclonal to vinculin (ab18058) were from Abcam.

### Cell lines and plasmids

U2OS, HeLa, and stable cell lines U2OS DR-GFP, U2OS EJ5-GFP, HeLa EJ5-GFP, U2OS SA-GFP and HeLa SA-GFP were grown in DMEM (Biowest, cat. L0104) supplemented with 10% FBS (Biowest, cat. S181B) and plasmocin 0.1 mg/l (Invivogen, cod ant-mpt). Primary fibroblasts were grown in DMEM supplemented with 20% FBS and plasmocin. I-SCEI-expressing plasmid pCBAS, empty vector (pCAGGS) and GFP-expressing plasmid NZE-GFP were kindly provided by Dr. Maria Jasin (Memorial Sloan Kettering Cancer Center, New York). EJ5-GFP (plasmid 44026) and SA-GFP (plasmid 41594) were from Addgene. For DNA and siRNA transfection lipofectamine 2000 (cat. 11668) and lipofectamine RNAiMax (cat. 13778) were used, respectively, from Invitrogen.

### siRNA transfection oligonucleotides

Oligonucleotides were purchased from Sigma with the following sequences: Luciferase 5'-CGU ACG CGG AAU ACU UCG A-3' (SEQ ID NO: 1); C53-1 5'-GGC AGG AGA UUA UAG CUC U-3' (SEQ ID NO: 2); C53-2 5'-GGU UCG GAA UGU CAA CUA U-3' (SEQ ID NO: 3); C53-3 5'-CCC UGA CAC UGC UUG AAU A-3' (SEQ ID NO: 4); FANCD2-1 5'-CCU CGA CUC AUU GUC AGU CAA CUA A-3' (SEQ ID NO: 5); FANCD2-2 5'-CCA UGU CUG CUA AAG AGC GUU CAU U-3' (SEQ ID NO: 6); FANCD2-3 5'-GGU GAU GGA UAA GUU GUC GUC UAU U-3' (SEQ ID NO: 7); BRCA2 5'-GGA UUA UAC AUA UUU CGC A-3' (SEQ ID NO: 8).

### Yeast-two-hydrid assay

The screens were carried out as previously described in Martrat et al Breast Cancer Res, doi: 10.1186/bcr2862. Briefly, the baits corresponded to protein family domains identified by Pfam and intrinsically disordered regions predicted by PONDR. Proteome-scale Y2H screens were carried out using the mating strategy and two different cDNA libraries as sources of prey, of human fetal brain or spleen (ProQuest; Life Technologies). Bait fragments were obtained using cDNAs derived from healthy lymphocytes and were subsequently cloned into the Gateway pDONR201 vector (Life Technologies). Baits were 5'-sequenced so that they were confirmed and open-reading frames were correct. Products were then transferred to the pPC97 yeast expression vector to be fused with the DNA-binding domain of Gal4. Constructs were transformed into the AH109 (Clontech) yeast strain for screens (Y187 mate strain) using selective medium lacking histidine and supplemented with 10 mM 3-amino-triazole (Sigma-Aldrich) to test the interaction-dependent transactivation of the HIS3 reporter. Baits were previously been examined for self-activation at 3-amino-triazole concentrations in the range 10 to 80 mM. Under these conditions, >10⁷ transformants were screened for each bait. Positive colonies were grown in selective medium for three cycles (10 to 15 days) to avoid unspecific cDNA contaminants, prior to PCR amplification and sequence identification of prey.

### Immunoprecipitation experiments

HEK 293T cells expressing empty vector or Flag-BRCA2 were treated with double thymidine block (10 mM) followed by a 9 hours release to enrich S-phase cells, or left untreated. Cells were lysed and subjected to immunoprecipitation (IP) with anti-Flag M2 antibody. Immunoprecipitates and whole cell lysates were immunoblotted (IB) with antibodies shown. In other series of experiments, HEK 293T cells were left untreated or synchronised to S-phase by treatment with 2 mM hydroxyurea for 18 hours. Cells were lysed and subjected to IP with anti-IgG or anti-BRCA2 antibody, followed by immunoblotting with antibodies shown above.

### Survival and clonogenic assays

For survival experiments, U2OS cells were transfected twice with luciferase, BRCA2 or C53 siRNA at a final concentration of 20-40 nM in 6-multiwell plates. 24 hours later 1x10⁵ cells were seeded in duplicate in 6-multiwell plates (or 35 mm dish plates for irradiation) and left untreated 24 hours more. Cultures were then exposed to mitomycin C (MMC), olaparib or γ-irradiated at the indicated doses. For survival assays, 72 to 96 hours after the treatment, cells were rinsed with PBS, harvested by trypsinization and counted. Survival is reported as the percentage relative to untreated controls. For clonogenic assays, cells were plated and treated with MMC or γ-irradiated at the indicated doses, left to grow for 10-14 days, Giemsa stained and colonies were finally counted.

### Cell cycle analysis

Cells from survival experiments were harvested, fixed in -20° C 70% EtOH and stored at -20° C. On the day of cell cycle analysis, cells were thawed, PBS rinsed, and incubated 30 min at 4° C in staining solution (propidium iodide 40 µg/ml and RNAse 100 µg/ml in PBS) and then analysed in a FACScalibur flow cytometer (Becton Dickinson).

### Sister chromatid exchanges (SCE)

Previous to SCE assay was done, U2OS cells were transfected twice with luciferase, BRCA2 or C53 siRNA in 6-multiwell plates. Twenty-four hours after the second transfection, cells were seeded in two 25 cm³ flasks. Next day, cells were treated with 26.05 µM of BrdU alone or with 0.01 µM of MMC. Cultures were maintained in total darkness to minimize BrdU photolysis. Forty-four hours later, colcemid (0.5 µg/ml) was added to all cultures and four hours later cells were harvested by trypsinization and diluted in a pre-warmed hypotonic solution (0.051 M KCl) for 25 min at 37 °C. Later cells were fixed in acetic acid:methanol solution (1:4 vol/vol). Fixed cells were dropped onto cleaned slides and left to age before staining with 1.56 µg/ml Hoechst and UVC irradiation (0.260 J/cm²) in 2xSSC buffer. After washing with 2xSSC, slides were stained with 10% Giemsa in phosphate buffer. SCE was analysed using a Zeiss Axio Observer A1 microscope.

### Chromosome fragility analysis

Primary fibroblasts were transfected twice with luciferase, BRCA2 or C53 siRNA in 10 cm dish plates. 24 hours after the second transfection, cells were replated to 5 cm dish plates and 24 hours later plates were irradiated at 0, 1 or 2 Gy. 24 hours after irradiation, colcemid was added at a concentration of 0.5 µg/ml. Cultures were harvested 6 h later when metaphase spreads were obtained and stained with Giemsa. 15-20 Metaphases with 46 chromosomes were analysed for each sample. The microscopic analysis was performed with a Zeiss Imager M1 microscope coupled to a computer-assisted metaphase finder (Metasystems, Altlussheim, Germany). The main criteria for the determination of chromosome fragility were as follows: chromatid and chromosome breaks were counted, figures were converted to the minimum number of breaks necessary to form each figure, and gaps were not counted as chromosome breaks.

### DNA repair assays

Homologous recombination (HR), single-strand annealing (SSA) and non-homologous end joining (NHEJ) assays were performed essentially as described (Pierce et al, 1999, Genes Dev.13: 2633-2638; Stark et al, 2004, Mol. Cell. Biol.24: 9305-9316; Bennardo et al, 2008, PLoS Genet. 4). For HR assay, U2OS DR-GFP stable cell line was used. For NHEJ and SSA assays, U2OS and HeLa cell lines were transiently transfected with EJ5-GFP or SA-GFP plasmids, selected with puromycin, and clonal cell lines obtained from limited dilution. Stable cell lines were confirmed to have specific GFP fluorescence upon I-SCEI but not upon empty vector transfection. For DNA repair assays, I-SCEI-endonuclease expressing vector, empty vector or GFP-expressing vector were transfected in U2OS or HeLa cell lines for DNA repair assays. Cells were transfected with NZE-GFP, pCBAS or pCAGGS for 16-24 hours with the indicated plasmids with Lipofectamine 2000 (Invitrogen) and 24 hours later collected and analysed by cell cytometry.

### Analysis of publicly available cancer transcriptomic datasets

To analyze the effect of C53 expression on the prognostics of ovarian and breast cancer patients, we generated Kaplan-Meier survival curves of these cancers with low or high expression of C53 by using Kaplan-Meier Plotter (www.kmplot.com) (Gyorffy et al, 2013, PLoS One 8*).*

### Results

### C53 interacts with BRCA2

The inventors screened for BRCA2 interactors by yeast two-hybrid screen with 5 BRCA2 bait proteins by Pfam-based search and predictor of naturally disordered regions (PONDR) algorithm (Martrat et al, 2011, Breast Cancer Res.13: R40), which covered most of the functionally important BRCA2 domains, such as the BRCT, helical, or OB domains (Fig 1A). Based on the helical and OB domain baits, 4 potential interactors were found: LRRC45, SIPA1L1, TACC3, and CDK5RAP3 (C53). Of these, the inventors focused on C53. The C53-BRCA2 interaction was confirmed by coimmunoprecipitation (coIP) in HEK293T cells. Cells were transfected with Flag-tagged BRCA2 and lysates immunoprecipitated with anti-Flag antibody, or untransfected cells immunoprecipitated with anti-BRCA2 antibody. As seen in Figure 1B and C, C53 interacted with BRCA2 in cells overexpressing Flag-BRCA2, as well as with endogenous BRCA2, only when cells were synchronised with double thymidine block or hydroxyurea treatment. These findings strongly suggest that BRCA2-C53 interaction occurs during the S-phase of the cell cycle.

### C53-depleted cells are resistant to a wide range of DNA damaging agents

The effects of C53 on the FA/BRCA pathway were also examined. In particular, the sensitivity of C53-depleted U2OS cells to DNA damage was compared with that of FANCD2- and BRCA2-depleted cells. RNA interference was used to inhibit C53, FANCD2, and BRCA2 and inhibition of all 3 target genes was confirmed by Western blot (Fig 2A and B). Survival and clonogenic assays after treating the cells with increasing doses of an interstrand cross-link DNA damaging agent (MMC), a double strand breaks (DSB) inducer (IR), or a PARP inhibitor (olaparib) was then performed. Inhibition of FANCD2 or BRCA2 rendered the cells highly sensitive to all the tested DNA damaging agents (Fig 2C-F). C53-depleted cells became resistant to MMC (Fig 2C and D), IR (Fig 2E), and olaparib (Fig 2F). Given that mutagen sensitivity depends on DNA replication and cell proliferation, the role of C53 in cell cycle progression was examined. The potential interference of cell growth in mutagen sensitivity was ruled out by the observation that cell population doubling of untreated C53-depleted cells was not affected, unlike that of FANCD2-depleted cells (Fig 8). In the analysis of cell cycle status, C53-depleted cells were not arrested in G2/M phase following MMC treatment. This finding was consistent with the increased resistance to MMC in the absence of C53 (Fig 8B and C), comparing with FANCD2-depleted cells. Finally, FANCD2 ubiquitination was analysed in these cells to check Fanconi Anemia (FA) pathway activation. While FANCD2 is not required for FANCD2 monoubiquitination, it was observed less FANCD2 activation in C53-depleted cells (Fig 8B and C), probably due to the lower proportion of cells in the S and G2/M phases.

### C53 regulates DSB repair and genomic stability

As BRCA2-deficient cells are defective in HR, it was examined whether C53 similarly regulated DSB repair. An *in vivo* HR assay was performed in U2OS cells by detecting the HR-dependent correction of an incomplete GFP expression cassette inserted in the genome (Fig 3A) in the presence or absence of C53 (Fig 3B) (Pierce et al, Genes Dev. 1999, 13: 2333-2638). Control cells with full HR repair pathway exhibited efficient functional GFP expression (Fig 3C bottom left panel). As expected, BRCA2 inhibition strongly abolished HR repair (Fig 3C bottom right and 3D). Interestingly, C53 downregulation in U2OS cells resulted in a 50% reduction in the percentage of fluorescent cells (Fig 3C bottom middle and 3D). Similar results were observed in a sister chromatid exchange assay, wherein HR-dependent exchanges of genetic material between sister chromatids were measured (Sonoda et al, 1999, Mol. Cell. Biol.19: 5166-9; Johnson & Jasin, 2000, EMBO J.19: 3398-3407) (Fig 3E).

The lack of HR repair in BRCA2-deficient cells is overcome by increasing error-prone repair via homology-directed single-strand annealing (SSA). To determine whether C53 plays a role in SSA, another *in vivo* cellular assay based in a SSA-dependent correction of an incomplete GFP expression cassette was performed (Fig 4A). Similar to the results of the HR assay, U2OS and HeLa cells with active SSA repair exhibited functional GFP expression (Fig 4B bottom left panel). As expected, BRCA2 depletion by siRNA transfection resulted in a 5- to 30-fold increase in the number of fluorescent cells (Fig 4B bottom right and 4C-D). Interestingly, C53 depletion also resulted in a 2- to 10-fold increase in the number of fluorescent U2OS and HeLa cells (Fig 4B bottom middle and 4C-D).

To complete analysing the role of C53 in DSB repair, a third GFP reporter-based cellular assay for non-homologous end joining in U2OS and HeLa cells was performed (Bennardo et al, 2008, PLoS Genet, 4) and found that depletion of C53 (or BRCA2) had little or no effect on non-homologous end joining (Fig 9).

A hallmark of BRCA2-defective cells is excessive chromosome instability upon treatment with chromosome-breaking agents. Therefore, spontaneous and ionizing radiation (IR)-induced chromosome fragility was quantified in the presence or absence of C53. As expected, BRCA2-depleted primary fibroblasts showed higher spontaneous and IR-induced chromosome fragility than control cells (Fig 5A and B). Interestingly, C53-depleted primary cells too showed increased spontaneous and IR-induced chromosome fragility, even though they were mutagen-resistant (Fig 2).

These findings suggest that C53 is involved in DNA repair by HR and SSA, similar to BRCA2, and that the genome instability in C53-depleted cells probably results from the use of error-prone SSA repair instead of high-fidelity HR repair.

### C53 expression modulates Survival rates of breast and ovarian cancer patients

Given that C53 interacts with BRCA2, mutations of which are strongly associated with breast and ovarian cancers, and that C53 downregulation leads to increased genome instability and mutagen resistance, it was hypothesized that C53 expression may be associated with reduced survival of patients with these tumours. To test this hypothesis, we used the Kaplan Meyer Plotter cancer transcriptomics database (Gyorffy et al, 2013, PLoS One 8). Analysis of tumour transcriptome data from 3,554 breast cancer patients revealed an association between low C53 expression and low overall survival (HR=0.62; P=0.00036) and relapse-free survival (HR=0.61; P<10⁻¹⁶, Fig 6 A, B). A similar association was observed in 388 patients with high-grade serous ovarian cancer (HGSOC, HR=0.72, P=0.03 for overall survival; HR=0.76, P=0.043 for progression-free survival, Fig 6C and D). An additional data analysis also showed an association between low survival rates and low C53 expression in basal, luminal A, luminal B, ER+, and ER- breast cancer patients, with the exception of the HER2+ subset, in which low C53 expression was associated with increased survival (HR=2.5, P=0.023 for overall survival, Fig 11 and 12). Together, these findings strongly suggest that C53 expression could act as a novel prognostic marker for breast and ovarian cancers.

## Claims

1. An *in vitro* method for determining the prognosis of a patient suffering from cancer, wherein the cancer is not hepatocarcinoma, comprising the steps of:
(a) determining the expression level of Cyclin-Dependent Kinase 5 Regulatory Subunit Associated Protein 3 (CDK5RAP3) gene in a sample from said patient, and
(b) comparing said expression level with a reference value,
wherein a decreased expression level of CDK5RAP3 gene compared to the reference value is indicative of a poor prognosis or
wherein an increased expression level of CDK5RAP3 gene or a lack of change compared to the reference value is indicative of a good prognosis.

2. The method according to claim 1, wherein said prognosis is **characterized by** predicting overall survival (OS) and/or progression free survival (PFS) and/or relapse free survival (RFS).

3. An *in vitro* method for predicting the clinical response of a patient suffering from cancer to a treatment with a DNA damaging agent comprising the steps of:
(a) determining the expression level of CDK5RAP3 gene in a sample from said patient and
(b) comparing said expression level with a reference value,
wherein a decreased expression level of CDK5RAP3 gene compared to the reference value is indicative of a poor clinical response of the patient to said treatment.

4. An *in vitro* method for selecting a patient suffering from cancer for a therapy with a DNA damaging agent, comprising the steps of:
(a) determining the expression level of CDK5RAP3 gene in a sample from said patient and
(b) comparing said expression level with a reference value,
wherein the patient is selected for said therapy if the expression level of CDK5RAP3 gene is not decreased compared to the reference value.

5. An *in vitro* method for selecting a therapy for a patient suffering from cancer, comprising the steps of
(a) determining the expression level of CDK5RAP3 gene in a sample from said patient and
(b) comparing said expression level with a reference value,
wherein if the expression level of CDK5RAP3 gene is not decreased compared to the reference value, a therapy with a DNA damaging agent is selected and wherein if the expression level of CDK5RAP3 gene is decreased compared to the reference value, an alternative therapy is selected.

6. The method according to any of claims 3 to 5, wherein the DNA damaging agent is selected from the group consisting of an interstrand-crosslink inducer, a double-strand break inducer and a PARP inhibitor.

7. The method according to claim 6, wherein the interstrand-crosslink inducer is mitomycin C, the double-strand break inducer is ionizing radiation and the PARP inhibitor is Olaparib or Veliparib.

8. The method according to claim 7, wherein the ionizing radiation is gamma radiation.

9. The method according to any of claims 3 to 8, wherein the cancer is osteosarcoma.

10. The method according to any of claims 1 to 9, wherein the cancer is breast cancer or ovarian cancer.

11. The method according to claim 10, wherein the cancer is a breast cancer selected from the group consisting of basal-like, luminal A, luminal B, ER+ and ER-breast cancer.

12. The method according to any of claims 10 or 11, wherein the cancer is not a HER2-positive breast cancer.

13. The method according to any of claims 1 to 12, wherein the sample is a tumor sample.

14. Use of a reagent capable of determining the expression level of CDK5RAP3 gene in a sample from a subject suffering from cancer for determining the prognosis of said patient, wherein the cancer is not hepatocarcinoma, or for predicting the clinical response of a patient suffering from cancer to a treatment with a DNA damaging agent, or for selecting a patient suffering from cancer for a therapy with a DNA damaging agent, or for selecting a therapy for a patient suffering from cancer.

15. A DNA damaging agent-based treatment for use in the treatment of cancer in a patient, wherein a sample of said patient shows increased or equal expression levels of CDK5RAP3 gene compared to a reference value.


SEQUENCE LISTING

<110> UNIVERSITAT AUTÒNOMA DE BARCELONA
FUNDACIÓ PRIVADA INSTITUT DE RECERCA BIOMÈDICA DE BELLVITGE
INSTITUT CATALÀ D'ONCOLOGIA

<120> PROGNOSTIC BIOMARKER FOR CANCER

<130> P12810EP00

<160> 8

<170> PatentIn version 3.5

<210> 1
<211> 19
<212> RNA
<213> Artificial Sequence

<220>
<223> luciferase siRNA

<400> 1
cguacgcgga auacuucga 19

<210> 2
<211> 19
<212> RNA
<213> Artificial Sequence

<220>
<223> C53 siRNA 1

<400> 2
ggcaggagau uauagcucu 19

<210> 3
<211> 19
<212> RNA
<213> Artificial Sequence

<220>
<223> C53 siRNA 2

<400> 3
gguucggaau gucaacuau 19

<210> 4
<211> 19
<212> RNA
<213> Artificial Sequence

<220>
<223> C53 siRNA 3

<400> 4
cccugacacu gcuugaaua 19

<210> 5

<211>  25
<212>  RNA
<213>  Artificial Sequence

<220>
<223>  FANCD2 siRNA 1

<400>  5
ccucgacuca uugucaguca acuaa                                              25


<210>  6
<211>  25
<212>  RNA
<213>  Artificial Sequence

<220>
<223>  FANCD2 siRNA 2

<400>  6
ccaugucugc uaaagagcgu ucauu                                              25


<210>  7
<211>  25
<212>  RNA
<213>  Artificial Sequence

<220>
<223>  FANCD2 siRNA 3

<400>  7
ggugauggau aaguugucgu cuauu                                              25


<210>  8
<211>  19
<212>  RNA
<213>  Artificial Sequence

<220>
<223>  BRCA2 siRNA

<400>  8
ggauuauaca uauuucgca                                                     19